# EUROPEAN PATENT SPECIFICATION

(11) **EP 2 910 633 B1**
(45) Date of publication and mention of the grant of the patent: **14.08.2019**
(21) Application number: 13847830.0
(22) Date of filing: 21.10.2013
(51) Int. Cl.: C12N 15/09, C12N 5/10, C12Q 1/02, C12Q 1/66, G01N 33/50

(54) **METHOD FOR EVALUATING BOTH INDUCTION OF DRUG-METABOLIZING ENZYME AND CYTOTOXICITY, AND VECTOR AND CELL FOR USE IN SAID METHOD**
VERFAHREN ZUR MESSUNG DER INDUKTION EINES METABOLISIERENDEN ENZYMS WIE AUCH DER ZYTOTOXIZITÄT SOWIE VEKTOR UND ZELLE ZUR VERWENDUNG IN DIESEM VERFAHREN
PROCÉDÉ POUR ÉVALUER À LA FOIS L'INDUCTION D'UNE ENZYME MÉTABOLISANT UN MÉDICAMENT ET LA CYTOTOXICITÉ, ET VECTEUR ET CELLULE DESTINÉS À ÊTRE UTILISÉS DANS LEDIT PROCÉDÉ

(30) Priority: 19.10.2012 JP 2012232018
(43) Date of publication of application: 26.08.2015
(73) Proprietor: National University Corporation Tottori University, Tottori-shi Tottori 680-8550 (JP)
(72) Inventor: TADA, Masako, Yonago-shi Tottori 683-8503 (JP); OSHIMURA, Mitsuo, Yonago-shi Tottori 683-8503 (JP); KAWAMURA, Fumihiko, Yonago-shi Tottori 683-8503 (JP); TSUJI, Saori, Yonago-shi Tottori 683-8503 (JP)
(74) Representative: Zwicker, Jörk
(86) International application number: PCT/JP2013/079058
(87) International publication number: WO 2014/061829

(56) References cited:
- WO-A1-02/24918
- WO-A1-2007/002568
- WO-A1-2011/083870
- JP-A- 2008 188 024
- US-A1- 2007 020 610
- TSUJI SAORI ET AL: "Dual-Color Fluorescence Imaging to Monitor CYP3A4 and CYP3A7 Expression in Human Hepatic Carcinoma HepG2 and HepaRG Cells", PLOS ONE, vol. 9, no. 8, August 2014 (2014-08), XP002758508,
- MATSUNAGA,T. ET AL.: 'Mechanisms of CYP3A induction by glucocorticoids in human fetal liver cells' DRUG METAB.PHARMACOKINET. vol. 27, no. 6, 22 May 2012, pages 653 - 657, XP055247626
- NEUNZIG,I,. ET AL.: 'Whole-cell biotransformation assay for investigation of the human drug metabolizing enzyme CYP3A7' BIOCHEM.BIOPHYS. ACTA vol. 1814, 2011, pages 161 - 167, XP027515070
- KAORI AIKAWA ET AL.: 'Hito Haisei Kansaibo Yurai Kansaibo ni Okeru CYP3A no Hatsugen to Yakubutsu ni yoru Yudo: Hito Taiji Kansaibo tono Hikaku' SEIKAGAKUKAI TAIKAI YOKOSHU 2008, page 2P-1164, XP008178924
- KAORU KOBAYASHI ET AL.: 'Yakubutsu Sogo Sayo Kenkyu eno Oyo o Mezashita Shinki Hito-gata CYP3A Cluster Donyu Mouse no Sakusei to Hyoka' DAI 23 KAI THE JAPANESE SOCIETY FOR THE STUDY OF XENOBIOTICS NENKAI KOEN YOSHISHU vol. 23RD, 2009, page 206, F-5, XP055248112
- USUI,T. ET AL.: 'Induction of CYP3As in HepG2 cells by several drugs.-Association between induction of CYP3A and Expression of glucocorticoid receptor' BIOL.PHARM.BULL. vol. 26, no. 4, 2003, pages 510 - 517, XP055247844
- TAMIHIDE MATSUNAGA ET AL.: 'Expression of cytochrome P450 in human fetus: focused on CYP3As in liver' THE SHINSHU MEDICAL JOURNAL vol. 56, no. 1, 2008, pages 7 - 16, XP055248104
- TETSUYA KAMATAKI: 'Function and its Application of Cytoclirome P450 : To Achieve Drug Metabolism Studies for Humans' XENOBIOTIC METABOLISM AND DISPOSITION vol. 13, no. 2, 1998, pages 173 - 181, XP055247852
- ISE,R. ET AL.: 'Expression of Cytochromes P450 in fetal, infant, and juvenile liver of Cynomolgus Macaques' DRUG METAB,PHARMACOKINET. vol. 26, no. 6, 2011, pages 621 - 626, XP055247873

### SEQUENCE LISTING

<110> A METHOD FOR EVALUATING A DRUG-METABOLIZING ENZYME INDUCTION AND A CELL TOXICITY, AS WELL AS A VECTOR AND A CELL THEREFOR.
<120> NATIONAL UNIVERSITY CORPORATION TOTTORI UNIVERSITY
<130> 671670
<150> JP 2012-232018
   <151> 2012-10-19
<160> 47
<170> Patent in version 3.2
<210> 1
   <211> 1012
   <212> DNA
   <213> Homo sapiens
<400> 1
   tcctgtttcc agacatgcag tatttccaga gagaaggggc cactctttgg caaagaacct      60
   gtctaacttt ctatctacgg caggactttt gaaagctaca gaggaagaag cacaaattga     120
   tgctattcca ctaagccatc agctccatct catccatgcc atgtctcttt tttaggggtc     180
   ctcttgccaa cagaatcaca gaggacaaat ctgaaagtgc agagacagca gctgaggcac     240
   agccaagagc tctggctgta ttaatgacct aagaagatgg agtggtcacc agaaagtcag     300
   aggaagtgac acacaggggc ccagcaatct cagccaagtc aactccacca gcctttctgg     360
   tccccactgt gtgtacagca ccctgatagg gaccagagcc atgagagtga gtaagaccag     420
   actatgccct tgaggagctc acctctgcta agggaaacag gcctggaaac acacaatggt     480
   ggtaaagagg aaagaagaca atagaactgc atgaagggga tggaaagtgc ccaggggagg     540
   aaatggttac ttctgtgtga gggggttggt gaggaaagac tctaagagaa ggctctgtct     600
   ggctgggtat gaaaggatgt gtaggagtct tctagggggc acaggcacac tccaggcata     660
   ggtaaagatc tgtaggcatg gcttgttggg atgagtttca agtattctgg aatgaggaca     720
   gccatagaga caagaggaga gttaatagat tttatgccaa tggctccact tgagtttgtg     780
   ataagaaccc agaacccttg gactccccag taacattgat tgagttgtgt atgattctac     840
   atagaatatt aactcaatgg aggtcagtga gtggtgtgtg tgtgattatt tgccaactgc     900
   cgaggtggag aagcctcttc cgactgcagg cagagcacgg gggccctgct actggctgca     960
   gctccagccc tgcctccttc tccagcatat aaacaatcca acagcctcac tg            1012
<210> 2
   <211> 123778
   <212> DNA
   <213> Homo sapiens
<400> 2
<210> 3
   <211> 11374
   <212> DNA
   <213> Homo sapiens
<400> 3
   ggatccctac tcactgtctt ctctctacag aacgagatgt ctctggagtc catagaaagc      60
   ccaggagcct ggctgggcac ggtggctcct gcctgtaatc ccagcacttt gggaggccga     120
   ggcaggcaga tcacctgagc tcaggagttc aagaccagcc tgggcaacat ggcaaaaccc     180
   catctctact aaaaatacaa aaaattagct gggcgtggtg gtgcatgcct ctaatcccag     240
   ctacttggga ggctgaggca caagaattgc ttgagcccag gaggcagcag ttgcagtgag     300
   ctgagattgt gccagtgcac tccagcctgg gcaacagagc aagattccat ttcaaaaaca     360
   aaaacaaaca caaacaaaca aacaaaaata gaaagcccag ggaccacctg cgtcaggttc     420
   ccagccacac ctttttcttg tcctcctctg tctctggcat cttctcacag gttcctaatt     480
   gtttgtggtt gcacaaattc aaaatcccag aaaaattacc acttcacacc cactcagatg     540
   gctatttttt ttttgaagga agataacaag tgttgacaag aacatggaga aattggaatt     600
   ctcacccatt gctggtgaga atgtaatacg gtgctgctgc tatggaaaac agcttggagt     660
   ttcctcaaaa agttcaacag aatttcaatg tgacccagca attcccctct aagttataga     720
   tctgagagga ttaaaaacag ttactaaaat acacggactc acatatttct aacagtccaa     780
   ttcacaaggg ccaaaaggtg ctaatagccc acatgtccat cgatggatgg ataaataaat     840
   tgtggtctat ccatacaatg gaatattatt cggccataaa tggaatgaag tactgacgca     900
   tgctacagaa tggatgaacc gcaaaaaaaa tggatgaaca catgctacag aatggatagc     960
   ctcactttac tatgaagtga aggccagaaa cgaagtccat atattgcatc atacaaaata    1020
   tccagaagag ggaagcccac agagacagaa tgtgcaatgg tggatgccag ggtctgggga    1080
   gaggggagag tggggagaaa ctgctcaact ggtacaggct ttattttgga atgatgggaa    1140
   cattttgcaa ctagatagag gtagtgattg cagaacacag aatgtactga attccactga    1200
   tttttttcac cttaaaatgg ttaattttca gtcctgagat tggataatca taaaaaaatg    1260
   gttaatttta tgttatgtga atttcatccc tatacatatt ttaaacctca gaaatataca    1320
   ctagcaggca tggaacaggt cactgtggtg cctgccaagc ccggtgatgt tatctggggt    1380
   ccccggccag ccttaagcct cttgctgacc ggtggagggc agaacctttg ccctaaaagt    1440
   ataatatcca catgctggca tgattcctgg ccagatggct tctttattag cagtaattga    1500
   aactgcctcg atacagacac tgtaccttgc aaccaaaaaa tgactcaaca atgataataa    1560
   gggttaagct gggcctttct ctctttgcca gttaaattat atttattata gcttgacatg 1620
   aaaaacaaag caactccaac aggtatcaca agggcaaagg acatgaacat tttatcaaag 1680
   aagaaatgca gctgtcaaaa atacagaaat attcaacctt gttcataata aagtggctgg 1740
   gctcagtggt tcatgcctgt aatcccagtg ctttgcaagg ctgagacagg aggatcattt 1800
   gaagccagaa gttcaagacc atcctaggca agtcagttca ataccagact tcatgtctac 1860
   aaaacatcaa aaaattagcc aggcatggtg atgcatgcct gttgtcccag ctactcagga 1920
   ggctgaggca ggagaattgc ttgagcctgg gaggctgcgg tggcggtgag ccatgattgt 1980
   gccattgtac tccagcctgg gcaatgcagc aagactgtct aaataacaaa aataatagta 2040
   aagaaaagga ttgggatgcc atttacttgc gtattcaata cacagagtta aaagtaattt 2100
   ctacgttttc tattttttta ttactaaaaa aagctggacc attctcacag cctgaaatgc 2160
   ttctcacttt cccttcttct gtccaaacac ttctctatga taatgcaaac agtcactcct 2220
   ttaggaagac ttcaccccag gtagttccag atccccttat ctctgccttc ccagaactcc 2280
   tggtgtctct ccagttccct ccgtgtggtg aagtacccta cctagggttt cagtatggct 2340
   ctgtctgcaa aggtcttgtt cacaccttcc cttatggttc tgttgccctg tgttgtgtca 2400
   tagcacaggg cacagtggag aacccattca cactgataga gagggcccca tggtcctgga 2460
   gataaccatg taaccgatca gaataaggca ttgagggctg ggtgtcaggc gtgggctgca 2520
   cttgggtggg caggtcccct ggaaagtcac tgggtttggc aagcttccta gtaacatgtc 2580
   tctctggggt cccccttgga acttcatgca aaaatgctgg ttgctggttt attctagaga 2640
   gatggttcat tcctttcatt tgattatcaa agaaactcat gtcccaatta aaggtcataa 2700
   agcccagttt gtaaactgag atgatctcag ctgaatgaac ttgctgaccc tctgctttcc 2760
   tccagcctct cggtgccctt gaaatcatgt cggttcaagc agcctcatga ggcattacaa 2820
   agtttaatta tttcagtgat tattaaacct tgtcctgtgt tgaccccagg tgaatcacaa 2880
   gctgaacttc tgacaagaac aagctatcat attcttttca attacagaaa aaagtaagtt 2940
   aattgatagg attttttttg tttaaaaaaa atgttactag ttttgaaaag gtaatatgtg 3000
   cacatggtaa acactaagaa ggtataagag cataatgctt ttatactact aagaataatg 3060
   ttttctctaa gttttttttg gtagatgctt tcatcagatt aagaaaattc cctgctatta 3120
   gttgttgaag gtttttatat cataaatgaa agttgaatat tattatcata tattattaat 3180
   atattgttat tgaactatca aagccttttc ctaaaaccat tgagatgatc ttataaccat 3240
   tctcctttaa cctgttgacg agatcattgg tatttatact atttctctgt taaccattct 3300
   tgagtctcag gtttaaattc aacttggtca tggtgtgtca tctttgatca ttgctgtctg 3360
   tggcttgcta ctgttttgtt taggattttt gcactgatgc tcatcaatga gactggcatg 3420
   ccatcttcct ttgcagtcct gatttttttc tgatttggat catgtggtta tggccctcat 3480
   ggaatgagtt gggcatgatg cctttttttc atgtctctgg attgatggga cactttggat 3540
   tctctccaga tggccctcaa tggtccctgc ctcctcattg ttaggcccct gggcaagccc 3600
   ttctcatttc tggtaggccc aggaacctgt gggggttttg tttgtttgtt tgtttcttga 3660
   gtcggagtct cactctgtca cccaggctgg agttggagtg caatggcccg atcttggctc 3720
   actgcaacct ccacctccca gattcaagca attctcctgc ctcagcctcc tgagtagctg 3780
   gaattacagg cacccaccga cacaccctgc taatttttgt atttttagta cagatggggt 3840
   ttcacaatat tggccaagct ggtctcgaac tcctgatctc atgatctgcc cggcttggcc 3900
   tcccaaagtg ttgagattac aagcatgagc caccacaccc agtgaacctg tggtttttag 3960
   aagctcccca tgcatgtgaa tgctgtgagc atcccaggat gacagccact gtgtgttcag 4020
   ctgttggaac tgtgagaaag caccagtggg accttctcca gcacctgcct gctgagttca 4080
   tggaagaggc ttgttgggga gatgatgccc tggctgactc ctgaaggatg gttaggaatg 4140
   caccagatgg aagctgggtt ggacccactc tatgctgaag aacagcttgt gtggacacaa 4200
   ggagacacgg atatgtcatt tttgtagagc ctgaggagtg tccaatcaca ccatttgctt 4260
   aaaacatcat gcacacttgg aaaagtggac tgagaccgaa tgaagaagct aacagtggcc 4320
   agatcagaaa gggtcttgtg ttacttccta gagatactta gattttatcc tgtgggtgat 4380
   aggagcagtt ggagggactg aagacaagga aagaaacatg tttcaagatc tatgtttttc 4440
   aagacgcttt tctggtggct gagtagggaa ttccctggat aagtcctgcc cagggtcagg 4500
   caaaacaagt tagggggtta ctgaaataag gagtatgaga aatggtgtag gttgtgctga 4560
   cgttttgtaa cacatctcat gatgatcttc atttccttca ctaatttcct gtttcattaa 4620
   ttcccttcca cgtgctcttc tgaaatttgc ctcacattct ctgatttctc ttttacctgt 4680
   tggtttcatc accttttact ttttgctttc ctggaaacac aaatgattct gattgtgaca 4740
   tgtcagaatt atttgcaaca tttgcctttc tgctgaaacc atgagttcac tgaatacaca 4800
   atttagtaaa gtgtaggatg cacatgtcgt tttcgtggtc acaaccagct ctgtagcatt 4860
   ttataactac actggcagtg tgctgggagg tgtagagaga aatatttatc acatgtgtgg 4920
   ctgacacaac ctgccaagtt attttaggag cctccttgga atcccagcaa gaatgctacc 4980
   ggcacaattt gtaatcacag catcctgctc catgccttgg cttcatggca tagtcacttc 5040
   tgcaagtctc tttccagctg tctgttccca tgtctataaa gtatgagtta aatcatccta 5100
   acactactca tcttacaaag ttttcttgct gatgttaaga gagttgggaa agaactgtat 5160
   aaactgtgaa gtgccatgga gatgttagtg gttactttat caagaaatag acactctaga 5220
   atggagtaga aagccaacag ttatgattga gtcctcctcc tcttcttctt tttattaatt 5280
   tataaagaaa agaggtttaa ttgactcaca gttccatatg gctggggagg cctcgggaaa 5340
   ctctcagtca tagcaggagg caaaggggaa gaaggcacct tcttcacaag gcggcaggag 5400
   agagagagct cctgttcttt tttgtcataa agtctacaga agtgcttata cttcaggaca 5460
   agggcaggca gagagaagga aggacattgc ttcaccccag ccctcactga cgagtttgct 5520
   aggggacctc actttgtccc agagtagggc agaactctgg ccactaccca ttcagaaggc 5580
   ctgggctgca ctgctagttc ctcactaact ctgtgtggcc ttgggcaagg ttgggcctgt 5640
   gttaacagat tatgaccctg ggctctcaag ctagaggatc taaatttgaa tcctggctct 5700
   gctaaagcaa ttagtgatgt aaactttaat gggtcagtta accttcctgt ggcttagttt 5760
   gctcatctgt aaaataggga tcataacagt atcaatacca catgattgtt ggacagattg 5820
   aatcagttaa tgcaggggaa gtacttagca tgacacgtat tcactatcat ttcctggagt 5880
   aagagctgtg tgtgagtggg tgtgagcatg tgtgaaacct tttctctgca atctcagtta 5940
   agaaaccaat ccagaattta aagttcaggg cctaaatggg tggttatctt ctcccagttc 6000
   catcctatcc cacctttgct cttcctcccg cccacaggag ctgttggtcc ttgattgggc 6060
   tggaagacct ggtggaccct aagtgatcta taagaggaga atagagaaca gggaatgtct 6120
   tcaaaaatct agagggacac agaggctgag aggcaggcag tcctgcaggg tcttctgatt 6180
   gggacaagga gaaccttggt cttcacaggc caattctggt cagtttcccc catggacaga 6240
   tgaggaaaca ggcccaggaa tatccaaggt ctcacacttc ccatctgtca agtcttgttg 6300
   attctgttgt attcatgtct ctcaaaggga gatagagttt agggaagaaa gaaggatcaa 6360
   ctgtgtctga taccactggg agcttaagta aagggttctt ttacttcata gcatttatcc 6420
   caatttgtaa ttcagtatta tttgtgtggc tgtttggtgt ctctttctcc tatatgagtg 6480
   ctagcttcat aagggcaagg attttgattc tttaatattt agtgcttgcc acatgccctg 6540
   aacacagcag gcatacaggc taaccaacat acagtggcat gaaagtcatg aaagtgagac 6600
   acctacctcc tccagtgcca agagagcata accatgcacc tgtcactctc ctcaacacca 6660
   cccccaagca tgaggcccaa aagcattagc taatcccctc ctccagccac taaaacttaa 6720
   aggccaggtg tggtggctcc catctgaaat cccagaactt caggagacag cagcaggagg 6780
   atcacttgag gccaggagtt tgagatcagc ctgggcaaca tagctaggtc ccatctgtac 6840
   taaaaattag ctgggcgttg ttgcatgcct gtagtcccag ctactaagga ggctgaggtg 6900
   ggaggatcac ttgagcccag gaggtggaaa caacagtaag ctataatcac agcactgaac 6960
   tctagcctgg gcaacagagt gacaccctgc ctcaaaacaa ttttaaaaat aaataagagc 7020
   aaaacttaga taccacgtgg tcaccccaac atgcaaaatc aagttttccc ctactgagaa 7080
   gaatggggac ttgacagctg agttacagag agataatctt cttcttcttt tttttttttt 7140
   ggtttacatc ctcaagatca tgacttgtga aatttgaatc gaatacacat gtaattccag 7200
   agcaatgttg cctccgcata ccatcagcaa ttcacttggc tactggaagt caggataagc 7260
   ttcccagaag agaggtacca cttgggctac caatataaaa ggatgaaaat atcagagtga 7320
   tggtgttctt tacaacgttg agtccctgga cagcctgtcc actgatgctg atatctgagc 7380
   ctaatgcttc tctgaatgtt gagattgaac tttgatccaa tgaaactaga acgagaaaga 7440
   agataagtct ttcattgttg ataaggacat tatgtttctc atacttgtat gattattttt 7500
   ccttagctgt actataatta tctgcttatt tgtctctgct ctatgtgctt agggtacaaa 7560
   gttgaccaag accaactttg gttggaagca tagtactaag agcacagtac tgagagcaca 7620
   gtattgagag cacagcttta aaaaacatga tgaaggcttt aatacaggaa atgagcaggg 7680
   gagaggcatg tggtggttgg atgtatcttc cttgacacag tcagtgcagc tctcagtagt 7740
   caagtcccta catgttagaa gatgttacct tctgtggaat taagtggcag aacttgcctt 7800
   caattatttt cctttgcaga acaacaccaa ctgcattagt taggacacag tgctggctgc 7860
   atttaagtcc caagcgatga ttagtctctc actgttggta tagattcaaa ccaatcagac 7920
   cacctcctaa agtttgtagg gcaggtaaat cctcatctta gaataaaaat catcttacca 7980
   agtatgtgtt ttagaggcaa gaagaaaaca tatttgtttc tgtaagagtt ttgtttaaaa 8040
   aaaatataag aaaggctctc ggtttaggtg aggtaatgaa gttgttgata gttatcagat 8100
   gacactggaa tctttacttc tctgaacgtg ttctgtgcat ctctcagtgt gggaacatag 8160
   agagggagat cctccagcaa tgccactgat atggtcagaa actgcatctt tctttctccc 8220
   tgctgagatg agatggagtc ctttgttcta gaagacccat ggtggtgccg ctgggagtaa 8280
   cccttgagac aggaacacaa atcccaacca atttgtggtt gcagccttga gtctcactat 8340
   ttcccatagt gatgcgtagc agggaatggc aggtgcacca gagcaggaga ggacctaata 8400
   tctcccttcc tgttagcttt ttataaagtt ttattgtgat cagtagcagt tgggaagcta 8460
   cttgcagtca ctgagcctca gtttctacat ctgtaaactg gggatagtag catggcccct 8520
   acttaatgtg ctcagcaaag ccactgaaag gagacagaaa tgtatctaaa ttaccctgga 8580
   cttttatcct acctctcttg gggattgtca ccaccttccc atgtttgtcc tttttggttt 8640
   gatgcttgct gtcacttctt tccttaggtg cctctctgta cggctctttt atcccaggga 8700
   ttccagagtt acagcacatg cataccacca tccaagcatg tttatttgtc tcctgcttca 8760
   ctaggctgtc cccaaggaac atgtggctcc cggcacacac ctggcacaac actgcacatg 8820
   acattcaccc acttggcctt gaatctgaca aggaatctgg catgatgttc acccactcag 8880
   gccaggtgcc gagcagccct ggaggcttag gggccagagg gatgggaaaa ggtgtctttc 8940
   tggggtgagt atcagtttct gcaggagggc tgaatgtgag aaagaataaa gagagaagga 9000
   agcgaacaag cacagcttaa acatcgccta tttctattga gttttaagaa cgctgtgatt 9060
   ttgtttgtca tgcaatccat tcatcaggcc aggcagacac agaacttggg tgtgagtgac 9120
   gataatgagc tgatataatt ttcacaccct catcactgag atctctccca tcaggaatgg 9180
   gtcagggagc tcacaggtgg cagcaactgc tattacaggc ctcatctcta ccagctcctg 9240
   gggcctgccc tcctcccatt agaaaatcct ccacttgtca aaaaggaagc catttgcttt 9300
   gaactccaat tccaccccca agaggctggg accatcttac tggagtcctt gatgctgtgt 9360
   gacctgcagt gaccactgcc ccatcattgc tggctgaggt ggttggggtc catctggcta 9420
   tctgggcagc tgttctcttc tctcctttct ctcctgtttc cagacatgca gtatttccag 9480
   agagaagggg ccactctttg gcaaagaacc tgtctaactt gctatctatg gcaggacctt 9540
   tgaagggttc acaggaagca gcacaaattg atactattcc accaagccat cagctccatc 9600
   tcatccatgc cctgtctctc ctttaggggt ccccttgcca acagaatcac agaggaccag 9660
   cctgaaagtg cagagacagc agctgaggca cagccaagag ctctggctgt attaatgacc 9720
   taagaagtca ccagaaagtc agaagggatg acatgcagag gcccagcaat ctcagctaag 9780
   tcaactccac cagcctttct agttgcccac tgtgtgtaca gcaccctggt agggaccaga 9840
   gccatgacag ggaataagac tagactatgc ccttgaggag ctcacctctg ttcagggaaa 9900
   caggcgtgga aacacaatgg tggtaaagag gaaagaggac aataggattg catgaagggg 9960
   atggaaggtg cccaggggag gaaatggtta catctgtgtg aggagtttgg tgaggaaaga 10020
   ctctaagaga aggctctgtc tgtctgggtt tggaaggatg tgtaggagtc ttctaggggg 10080
   cacaggcaca ctccaggcat aggtaaagat ctgtaggtgt ggcttgttgg gatgaatttc 10140
   aagtattttg gaatgaggac agccatagag acaagggcaa gagagaggcg atttaataga 10200
   ttttatgcca atggctccac ttgagtttct gataagaacc cagaaccctt ggactcccca 10260
   gtaacattga ttgagttgtt tatgatacct catagaatat gaactcaaag gaggtcagtg 10320
   agtggtgtgt gtgtgattct ttgccaactt ccaaggtgga gaagcctctt ccaactgcag 10380
   gcagagcaca ggtggccctg ctactggctg cagctccagc cctgcctcct tctctagcat 10440
   ataaacaatc caacagcctc actgaatcac tgctgtgcag ggcaggaaag ctccatgcac 10500
   atagcccagc aaagagcaac acagagctga aaggaagact cagaggagag agataagtaa 10560
   ggaaagtagt gatggctctc atcccagact tggccatgga aacctggctt ctcctggctg 10620
   tcagcctggt gctcctctat ctgtgagtaa ctgtccaggc tcctcttctc tgtttccttg 10680
   gacttggggt gctaatcagg cctctctttc ccttatctgt tttgaagatc aaaaaagatg 10740
   ttcaggccgg gcgtggtggc ttacacctgt aatcccagca ctttgggagg ctaaggcaag 10800
   tggactgcct gaggtcagga gttcaagacc agcctggcta acatggtgaa actctgtctc 10860
   tactaaaaat acaaaaatta gctgggcatg gtggtgcacg cctgtattcc cagctacttg 10920
   ggaggctgag gcaggagaat tgcttgaacc cggcaggcgg aggttgcagt gagctgagat 10980
   catgccagtg cacttcagcc tgggtgacag agtgaggctg tctcaaaaaa aaaaaaaaaa 11040
   aaaaaaagat gttcaaggag cagtagctta agtgttggat gctacaaaca tatagaggtt 11100
   attgtagatc ttatgcagct ctataaagga ataaataagc atcttcccca tccatcttta 11160
   gtggcaagaa gggttttggg atagcattga ttgaggatga tctacttgac aatagtttgg 11220
   acccaaggag gataaggaag gaaagtagtg acggatctca ttccaaactt ggctgtggaa 11280
   acctggcttc tccttactaa actagaattt ggattttaca ttttcccctt tatgttgcag 11340
   tagaagagga tgaatcctct cactggtggg atcc 11374
<210> 4
   <211> 28
   <212> DNA
   <213> Artificial
<220>
   <223> primer
<400> 4
   caaagttgac caagaccaac tttggttg 28
<210> 5
   <211> 22
   <212> DNA
   <213> Artificial
<220>
   <223> primer
<400> 5
   ctgtctctgc actttcaggc tg 22
<210> 6
   <211> 23
   <212> DNA
   <213> Artificial
<220>
   <223> primer
<400> 6
   caacagaatc acagaggacc agc 23
<210> 7
   <211> 30
   <212> DNA
   <213> Artificial
<220>
   <223> primer
<400> 7
   cgtcactact ttccttcctt atcctccttg 30
<210> 8
   <211> 26
   <212> DNA
   <213> Artificial
<220>
   <223> primer
<400> 8
   ccagacaggg gactcagccc tgaata 26
<210> 9
   <211> 20
   <212> DNA
   <213> Artificial
<220>
   <223> primer
<400> 9
   ttgcaagcca cccctctgcg 20
<210> 10
   <211> 22
   <212> DNA
   <213> Artificial
<220>
   <223> primer
<400> 10
   caagcagaga aggggtcgac gc 22
<210> 11
   <211> 26
   <212> DNA
   <213> Artificial
<220>
   <223> primer
<400> 11
   ttagctgggg tgagatgcta tctcac 26
<210> 12
   <211> 30
   <212> DNA
   <213> Artificial
<220>
   <223> primer
<400> 12
   cttggaatcc cagcaagaac accactgatg 30
<210> 13
   <211> 28
   <212> DNA
   <213> Artificial
<220>
   <223> primer
<400> 13
   cctctgactt tctggtgacc actccatc 28
<210> 14
   <211> 30
   <212> DNA
   <213> Artificial
<220>
   <223> primer
<400> 14
   gctgtattaa tgacctaaga agatggagtg 30
<210> 15
   <211> 26
   <212> DNA
   <213> Artificial
<220>
   <223> primer
<400> 15
   tagtgacagc cccacaacat ggccag 26
<210> 16
   <211> 30
   <212> DNA
   <213> Artificial
<220>
   <223> primer
<400> 16
   gggttcccat gaatttgtct caggtcaaac 30
<210> 17
   <211> 28
   <212> DNA
   <213> Artificial
<220>
   <223> primer
<400> 17
   tgctcttgta ggccacacct ctctatga 28
<210> 18
   <211> 23
   <212> DNA
   <213> Artificial
<220>
   <223> primer
<400> 18
   gcaagcagag aaggggttca tag 23
<210> 19
   <211> 25
   <212> DNA
   <213> Artificial
<220>
   <223> primer
<400> 19
   ggctcactgc cacctatgcc tcatg 25
<210> 20
   <211> 21
   <212> DNA
   <213> Artificial
<220>
   <223> primer
<400> 20
   cagctcctcg cccttgctca c 21
<210> 21
   <211> 20
   <212> DNA
   <213> Artificial
<220>
   <223> primer
<400> 21
   gtagaaaaga tcaaaggatc 20
<210> 22
   <211> 24
   <212> DNA
   <213> Artificial
<220>
   <223> primer
<400> 22
   atggtgagca agggcgagga gctg 24
<210> 23
   <211> 21
   <212> DNA
   <213> Artificial
<220>
   <223> primer
<400> 23
   gcctcttcgc tattacgcca g 21
<210> 24
   <211> 21
   <212> DNA
   <213> Artificial
<220>
   <223> primer
<400> 24
   gatgacgtcc tcggaggagg c 21
<210> 25
   <211> 20
   <212> DNA
   <213> Artificial
<220>
   <223> primer
<400> 25
   gccttcttga cgagttcttc 20
<210> 26
   <211> 21
   <212> DNA
   <213> Artificial
<220>
   <223> primer
<400> 26
   gcctcctccg aggacgtcat c 21
<210> 27
   <211> 28
   <212> DNA
   <213> Artificial
<220>
   <223> primer
<400> 27
   gaagaactcg tcaagaaggc gatagaag 28
<210> 28
   <211> 22
   <212> DNA
   <213> Artificial
<220>
   <223> primer
<400> 28
   cgccgtccag ctcgaccagg at 22
<210> 29
   <211> 21
   <212> DNA
   <213> Artificial
<220>
   <223> primer
<400> 29
   ctgagatagg tgcctcactg a 21
<210> 30
   <211> 27
   <212> DNA
   <213> Artificial
<220>
   <223> primer
<400> 30
   attttcctaa ggacttctgc tttgctc 27
<210> 31
   <211> 22
   <212> DNA
   <213> Artificial
<220>
   <223> primer
<400> 31
   gccaacagtg attacaatga cc 22
<210> 32
   <211> 30
   <212> DNA
   <213> Artificial
<220>
   <223> primer
<400> 32
   tcacttcgca gtgccgcaag ccaaaggcaa 30
<210> 33
   <211> 30
   <212> DNA
   <213> Artificial
<220>
   <223> primer
<400> 33
   gcaggcatgc aagcttggcg taatcatggt 30
<210> 34
   <211> 30
   <212> DNA
   <213> Artificial
<220>
   <223> primer
<400> 34
   ccgaaaggcc caacaggcaa gctgatgaga 30
<210> 35
   <211> 30
   <212> DNA
   <213> Artificial
<220>
   <223> primer
<400> 35
   aacttctgtg cttaaaacgt catctgcatc 30
<210> 36
   <211> 23
   <212> DNA
   <213> Artificial Sequence
<220>
   <223> primer
<400> 36
   ttcatccaat ggactgcata aat 23
<210> 37
   <211> 28
   <212> DNA
   <213> Artificial Sequence
<220>
   <223> primer
<400> 37
   tcccaagtat aacactctac acagacaa 28
<210> 38
   <211> 23
   <212> DNA
   <213> Artificial Sequence
<220>
   <223> primer
<400> 38
   ttcatccaat gtgctgcata aat 23
<210> 39
   <211> 28
   <212> DNA
   <213> Artificial Sequence
<220>
   <223> primer
<400> 39
   taccaagtat aacactctat acagacca 28
<210> 40
   <211> 20
   <212> DNA
   <213> Artificial Sequence
<220>
   <223> primer
<400> 40
   cttcaaggag gacggcaaca 20
<210> 41
   <211> 20
   <212> DNA
   <213> Artificial Sequence
<220>
   <223> primer
<400> 41
   ccttgatgcc gttcttctgc 20
<210> 42
   <211> 19
   <212> DNA
   <213> Artificial Sequence
<220>
   <223> primer
<400> 42
   gaagggcgag atccacaag 19
<210> 43
   <211> 21
   <212> DNA
   <213> Artificial Sequence
<220>
   <223> primer
<400> 43
   ggacttgaac tccaccaggt a 21
<210> 44
   <211> 20
   <212> DNA
   <213> Artificial Sequence
<220>
   <223> primer
<400> 44
   accatgaagt gggtggaatc 20
<210> 45
   <211> 20
   <212> DNA
   <213> Artificial Sequence
<220>
   <223> primer
<400> 45
   tggtagccag gtcagctaaa 20
<210> 46
   <211> 18
   <212> DNA
   <213> Artificial Sequence
<220>
   <223> primer
<400> 46
   attggcaatg agcggttc 18
<210> 47
   <211> 19
   <212> DNA
   <213> Artificial Sequence
<220>
   <223> primer
<400> 47
   ggatgccaca ggactccat 19

## Description

### Technical Field

The present patent application is a patent application regarding the results of a national government-commissioned research (Year 2010, Ministry of Education, Culture, Sports, Science and Technology, Innovation System Improvement Project, a commissioned research regarding "Formation of Chromosome Engineering Research Center associated with Drug Discovery, Development of Food Functionality Evaluation Model Animals and the like," a patent application under Section 19 of the Industrial Technology Enhancement Act).

The present invention relates to a method for evaluating both induction of a drug-metabolizing enzyme by a test substance and cytotoxicity of the test substance, and a vector and a cell for use in said method.

### Background Art

The major three conditions for the usefulness of a drug are "having an effect," "having low toxicity," and "not accumulated in a body." Regarding pharmaceutical product candidate compounds, absorption, distribution, metabolism, excretion, and toxicity are generally studied.

It has been known that the metabolism of a drug is carried out by various drug-metabolizing enzymes. Cytochrome P450, a representative drug-metabolizing enzyme, is an oxidase involved in 80% of drug metabolism in a living body. The CYP3A group includes major genes belonging to a cytochrome P450 family, and it is considered that the CYP3A is involved in the metabolism of approximately 50% of commercially available drugs (Non Patent Document 1).

With regard to drug-metabolizing enzymes, a phenomenon that is induction of a drug-metabolizing enzyme has been known. The induction of a drug-metabolizing enzyme is a phenomenon in which expression of a drug-metabolizing enzyme is increased by a specific drug through activation of a promoter of the drug-metabolizing enzyme and the like. Such induction of a drug-metabolizing enzyme causes a harmful drug interaction. For instance, when a first pharmaceutical agent that increases the expression of a drug-metabolizing enzyme and a second pharmaceutical agent that is different from the first pharmaceutical agent are simultaneously administered to a subject, the drug-metabolizing enzyme whose expression has been increased by the first pharmaceutical agent metabolizes the second pharmaceutical agent, and as a result, the medicinal effects of the second pharmaceutical agent are reduced. Accordingly, upon obtaining a useful drug, it is important to examine the induction of a drug-metabolizing enzyme by pharmaceutical product candidate compounds.

As a method for examining induction of a drug-metabolizing enzyme, there is a method which comprises: allowing a biological model cell comprising a vector in which a reporter gene is connected downstream of the expression control region of a drug-metabolizing enzyme gene to come into contact with a drug candidate substance; examining a change in the expression of the reporter gene in the cell between before and after the contact; and evaluating the drug candidate substance that has increased the expression of the reporter gene as a drug-metabolizing enzyme inducible substance (see Patent Documents 1 to 4). This method does not require complicated operations and can be easily carried out. However, some drug candidate substances that have been evaluated to cause induction of a drug-metabolizing enzyme by the aforementioned method do not actually cause the induction of a drug-metabolizing enzyme in living bodies. In contrast, some drug candidate substances that have been evaluated not to cause the induction of a drug-metabolizing enzyme actually cause it in living bodies. As such, conventional evaluation methods have been insufficient to ensure accuracy.

Patent Document 3 describes a vector having the expression region of CYP3A4 and a fluorescent protein. However, Patent Document 3 contains neither a description regarding the improvement of the performance of a cell with which a drug candidate substance is allowed to come into contact, nor a description regarding a fetus-specific gene.

Patent Document 4 describes the use of a CYP3A4 regulatory nucleic acid molecule and a reporter nucleic acid molecule, and contains descriptions regarding Examples in which β-galactosidase was used as such a reporter nucleic acid molecule. However, Patent Document 4 contains neither a description regarding Examples in which a fluorescent protein was used, a description regarding the improvement of the performance of a cell with which a drug candidate substance is allowed to come into contact, nor a description regarding a fetus-specific gene.

As a technique similar to the aforementioned techniques, Patent Document 5 discloses a method for evaluating the toxicity or bioavailability of a drug, using a vector having a fluorescent protein GFP as a reporter gene and comprising the promoter region of CYP3A7 that is a fetus-specific gene. However, the technique of Patent Document 5 is not intended to evaluate induction of a drug-metabolizing enzyme. Moreover, Patent Document 5 contains neither a description regarding a vector comprising a combination of the expression regions of multiple genes, nor a description regarding the improvement of the performance of a cell with which a drug candidate substance is allowed to come into contact.

### [Citation List]

### Patent Document

Patent Document 1: Japanese Patent Laid-Open No. 2008-546417
Patent Document 2: Japanese Patent Laid-Open No. 2005-229967
Patent Document 3: Japanese Patent Laid-Open No. 2003-304896
Patent Document 4: Japanese Patent Laid-Open No. 2010-148508
Patent Document 5: International Publication WO 02/24918

### Non Patent Document

Non Patent Document 1: Wilkinson, G.R. (2005). N Engl J Med 352, 2211-2221.

### Summary of Invention

### Technical Problem

Accordingly, it is an object of the present invention to evaluate the induction of a drug-metabolizing enzyme by a test substance more accurately than ever before, by simple operations, using a vector in which a reporter gene is connected downstream of the expression control region of a drug-metabolizing enzyme gene and a cell into which the aforementioned vector is introduced.

### Solution to Problem

As a result of intensive studies, the present inventors have found that a major cause of inaccuracy in conventional methods is that a cell population used in the evaluation is not homogeneous and the cell population contains a large number of cells that do not cause the same induction of a drug-metabolizing enzyme as that in a living body. In addition, the inventors have also found that another cause of inaccuracy is that the expression of a reporter gene has not been sufficiently functioned in cells for evaluation, when a test substance having cytotoxicity has been used. Moreover, in conventional methods, the expression level of a reporter gene has been observed only at a specific time point after the contact with a drug, and induction of a drug-metabolizing enzyme has been evaluated. The present inventors have found that the expression level of a reporter gene by induction of a drug-metabolizing enzyme may be reduced after it has been once increased, and thus that evaluation of induction of a drug-metabolizing enzyme based on the results of the expression level after it has been reduced would be another cause of inaccuracy in conventional methods. Furthermore, the inventors have confirmed that the expression of a fetus-specific gene transiently increases when a cell is damaged by a cytotoxic substance, and thus that it becomes an indicator of the cytotoxicity of a test substance.

Based on the aforementioned findings, the present inventors have conducted further studies. As a result, the inventors have found that a cell showing high maturity suitable for an accurate drug-metabolizing enzyme induction test can be selected by introducing a vector comprising an expression control region of a drug-metabolizing enzyme gene and an expression control region of a fetus-specific gene into cells, and then by selecting cells in which the expression level of a reporter gene located downstream of the expression control region of the fetus-specific gene is low. Moreover, the inventors have also found that if such a vector is used, induction of a drug-metabolizing enzyme can be evaluated by evaluating a change in the expression level of the reporter gene located downstream of the expression control region of the drug-metabolizing enzyme gene between before and after the contact of the cell with the test substance, and further, cytotoxicity can also be evaluated by evaluating a change in the expression level of the reporter gene located downstream of the expression control region of the fetus-specific gene between before and after the contact of the cell with the test substance, so that induction of a drug-metabolizing enzyme can be evaluated more accurately than ever before based on the evaluation of cytotoxicity. When the aforementioned vector was used, both selection of cells suitable for the drug-metabolizing enzyme induction test and a test of evaluating induction of a drug-metabolizing enzyme and cytotoxicity could be carried out by simple one-step operations, and the developmental stage and quality of cells were simultaneously evaluated so that the reliability of the obtained data could be confirmed.

In particular, when a reporter gene detected by light signal is used as a reporter gene located downstream of each expression control region, the expression level of the reporter gene could be evaluated according to live imaging, simply continuously and qualitatively, without analyzing a cell extract. Accordingly, even in a case where the expression was reduced after it had been increased by induction of a drug enzyme, the drug enzyme induction test could be carried out simply and accurately, without missing the process in which the expression had been increased. Furthermore, if the vector of the present invention comprising a reporter gene detected by light signal is used, immobilization and destruction of cells and addition of a substrate, as in luciferase or β-galactosidase staining, become unnecessary, and a continuous measurement becomes possible. Thus, it has been economically advantageous.

Further, the present inventors have also found that when a hepatoma-derived cell line is used as a cell into which the vector is to be introduced, since a change in the expression level of a drug-metabolizing enzyme is extremely clearly observed depending on a difference in the maturity of cells, cells with high maturity suitable for the test can be selected more reliably and simply.

Specifically, the present invention provides a vector for producing a cell that is used for evaluation of induction of a drug-metabolizing enzyme by a test substance and cytotoxicity of the test substance, which comprises an expression control region of a fetus-specific gene, a first reporter gene, an expression control region of a drug-metabolizing enzyme gene, and a second reporter gene, wherein the first reporter gene is located downstream of the expression control region of the fetus-specific gene, and the second reporter gene is located downstream of the expression control region of the drug-metabolizing enzyme gene,
wherein the drug-metabolizing enzyme gene is CYP3A4 and
the fetus-specific gene is CYP3A7.

Furthermore, the present invention provides the above described vector, wherein the reporter gene is detected by light signal.

Further, the present invention provides the above described vector, wherein the reporter gene encodes a fluorescent protein.

Further, the present invention provides a cell used for evaluation of induction of a drug-metabolizing enzyme by a test substance and cytotoxicity of the test substance, wherein the cell comprises the above described vector.

Further, the present invention provides the above described cell, which is derived from a hepatoma-derived cell line.

Still further, the present invention provides the above described cell, which has properties of a stem cell capable of differentiating into a mature hepatocyte.

Still further, the present invention provides the above described cell, which is selected by a method comprising:
(1) a step of culturing cells containing the above described vector; and
(2) a step of selecting a cell using an expression level of the first reporter gene in the cells during the culture in the step (1) as an indicator.

Still further, the present invention provides the above described cell, wherein the culture in the step (1) is a culture for allowing cells containing the above described vector to differentiate into hepatocytes.

In addition, the present invention provides a non-human animal comprising the above described vector.

Moreover, the present invention provides a method for evaluating induction of a drug-metabolizing enzyme by a test substance and cytotoxicity of the test substance, which comprises:
(a) a step of evaluating expression of the first reporter gene in the above described cell and then removing cells involving high expression of the first reporter gene from the cells used for evaluation, wherein the contacted test substance has cytotoxicity when the increase in the expression is observed; and
(b) a step of evaluating a change in expression of the second reporter gene in the above described cell before and after the contact of the above described cell with the test substance, comprising a step of determining that the contacted test substance has ability to induce a drug-metabolizing enzyme, when the expression of the second reporter gene is increased by the contact with the test substance,
wherein the first reporter gene is located downstream of the expression control region of the fetus-specific gene, and the second reporter gene is located downstream of the expression control region of the drug-metabolizing enzyme gene, wherein the drug-metabolizing enzyme gene is CYP3A4 and the fetus-specific gene is CYP3A7.

Furthermore, the present invention provides the above described method, wherein the evaluation of a change in the expression of the second reporter gene in the step (b) is based on the continuous measurement results of the expression of the second reporter gene.

Further, the present invention provides a kit for evaluating induction of a drug-metabolizing enzyme by a test substance and cytotoxicity of the test substance, wherein the kit comprises the above described vector.

Still further, the present invention provides the above described kit, further comprising a cell having properties of a stem cell capable of differentiating into a mature hepatocyte.

### Advantageous Effects of Invention

Using the vector of the present invention, a cell population used for simultaneously and accurately evaluating induction of a drug-metabolizing enzyme and cytotoxicity can be easily prepared. Upon preparation of such a cell population, the expression of a first reporter gene located downstream of the expression control region of a fetus-specific gene is evaluated, and if cells having a low expression level were selected, a cell population suitable for more accurate evaluation could be prepared. Moreover, since the vector of the present invention comprises both the expression control region of a drug-metabolizing enzyme gene and the expression control region of a fetus-specific gene, cells into which both of them are introduced at a constant ratio can be prepared using such a vector. With regard to the thus prepared cells, on the basis of the expression level of a reporter gene located downstream of a drug-metabolizing enzyme gene and the expression level of a reporter gene located downstream of a fetus-specific gene, the influence on cytotoxicity and the influence on the drug-metabolizing enzyme can be quantitatively evaluated. Furthermore, taking into consideration determination regarding whether or not the non-expression of the reporter gene is caused by the cytotoxicity of a test substance, the induction of a drug enzyme by the test substance can be evaluated more accurately.
Further, using the vector of the present invention, the developmental stage or quality of cells can be evaluated, and as a result, cells with high maturity suitable for a drug-metabolizing enzyme induction test can be selected more reliably and easily. For example, using the vector of the present invention, a developmental stage, in which stem cells in the liver, cell lines similar thereto, induced pluripotent stem (iPS) cells, embryonic stem (ES) cells and the like differentiate into functional hepatocytes, can be monitored, and the differentiated cells can be easily selected. Whether the cells suitable for evaluation of induction of a drug-metabolizing enzyme are developed by direct differentiation from hepatocytes during the fetal period or the cells are developed from other stem cells after the birth has been unknown. This phenomenon can be clarified by pursuing whether or not the cell of the present invention expressing a first reporter gene located downstream of the expression control region of a fetal hepatocyte-specific gene can directly differentiate into a cell suitable for evaluation of induction of a drug-metabolizing enzyme over time using a second reporter gene in the vector of the present invention and also using a single cell. It has been an object to develop a cell differentiation induction method for producing cells suitable for evaluation of drug-metabolizing enzyme inducibility from stem cells such as ES cells or iPS cells. The development of such a differentiation induction method can be accelerated by clarifying the developmental stage of a hepatocyte having high metabolic function by the expression switching of a reporter gene in the vector of the present invention. Hence, according to the present invention, a vector capable of evaluating drug-metabolizing enzyme inducibility, expression switching and hepatotoxicity, and a cell prepared by introducing the above described vector into a biological model cell capable of being used for such evaluation can be provided.

### Brief Description of Drawings

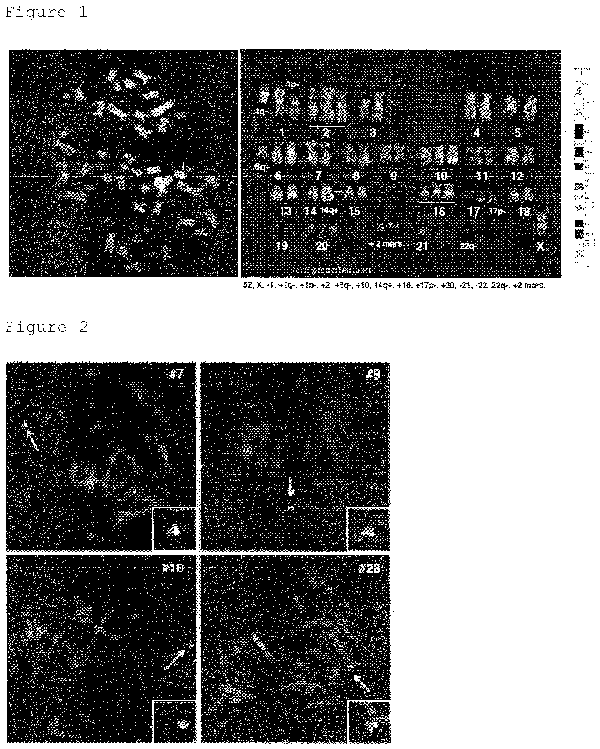

Figure 1 includes photographs showing a loxP-inserted site in loxP site-introduced HepG2 cells.
Figure 2 includes photographs showing confirmation of a BAC vector {CYP3A4+/7R BAC} comprising an entire-length human CYP3A4 gene and a red fluorescence reporter gene (R: DsRed) inserted downstream of the gene expression control region of a human CYP3A7 gene, which has been mounted on a mouse artificial chromosome vector MAC in CHO cells.

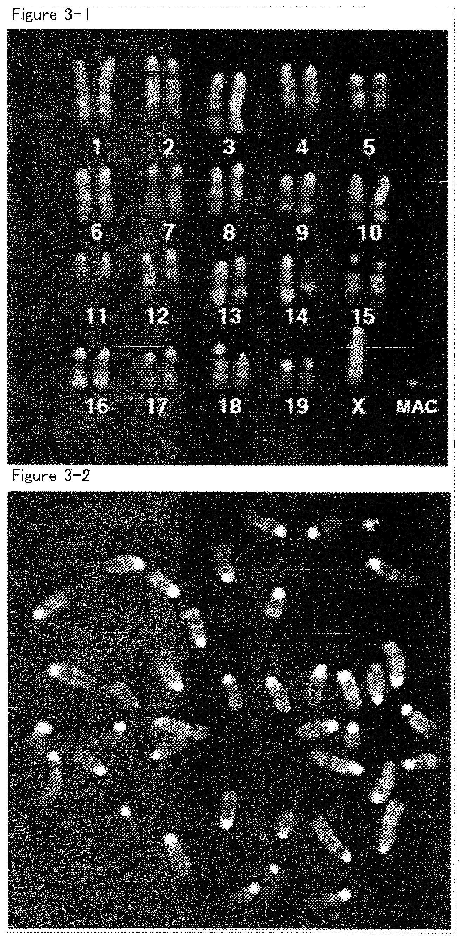

Figure 3-1 and Figure 3-2 are photographs showing the karyotype of CYP3A4+/7R BAC-mounted MAC{CYP3A4+/7R MAC}-introduced mouse ES cells (TT2F) (Figure 3-1), and an FISH analysis image in which the introduction of MAC has been confirmed (Figure 3-2).

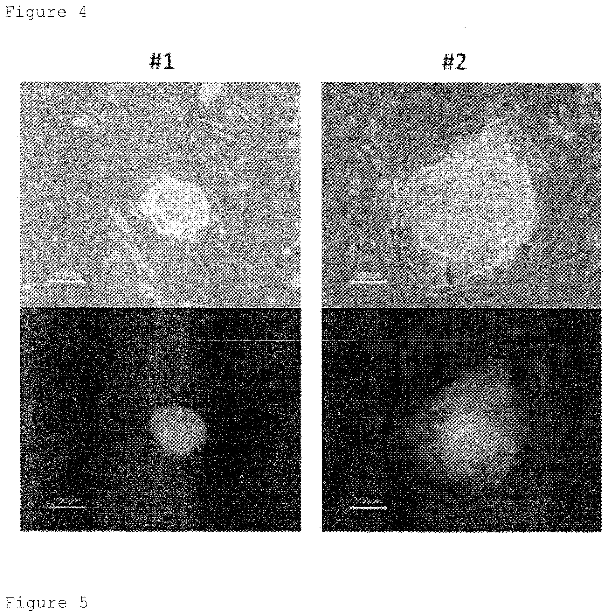

Figure 4 includes photographs showing colonies of CYP3A4+/7R MAC-introduced mouse ES cells. The expression of an EGFP gene that has previously been mounted on MAC can be confirmed.
Figure 5 includes photographs showing confirmation of the expression of liver-specific DsRed in chimeric baby mice (Day 1) using CYP3A4+/7R ES cells.

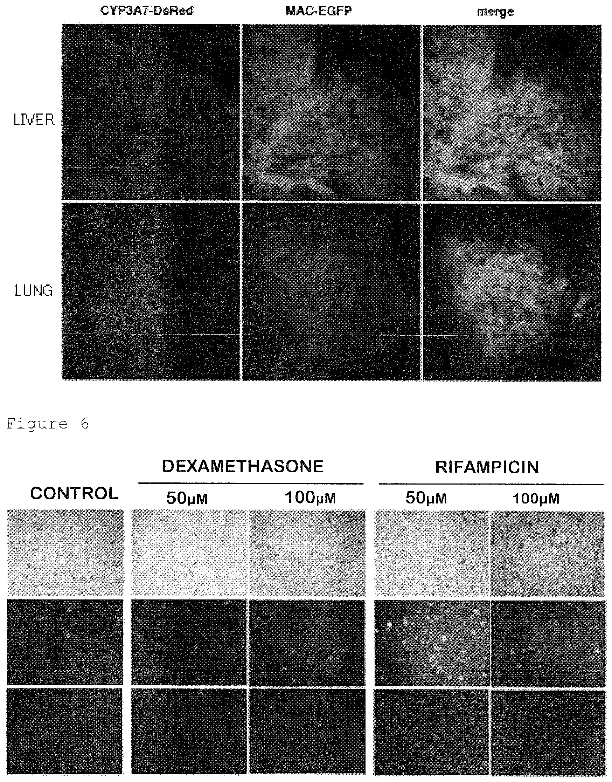

Figure 6 includes fluorescence microphotographs, in which 48 hours after addition of CYP3A4 and CYP3A7 (CYP3A4/7) expression-inducing drugs, the inducibility of the expression of BAC vector {CYP3A4G/7R BAC}-introduced HepG2 cells, wherein, in the vector, a green fluorescence reporter gene (G: EGFP) has been inserted downstream of the gene expression control region of the human CYP3A4 gene and a red fluorescence reporter gene (R: DsRed) has been inserted downstream of the gene expression control region of the human CYP3A7 gene, is evaluated based on the expression of the two fluorescence reporter genes.

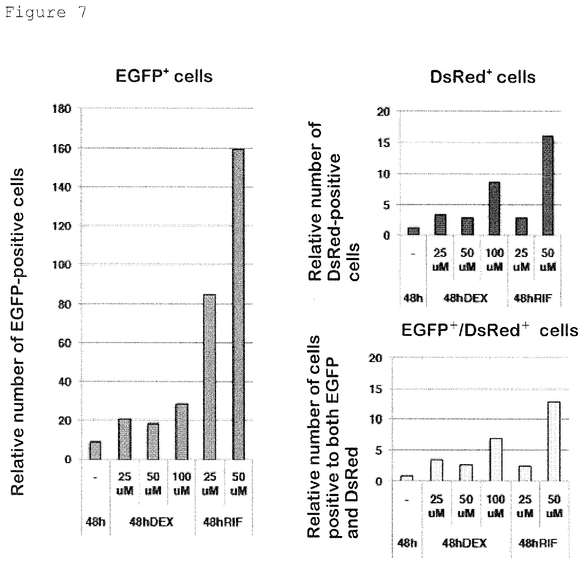

Figure 7 includes graphs showing the FACS analysis results obtained by evaluating the inducibility of the expression of CYP3A4G/7R BAC-introduced HepG2 cells 48 hours after addition of a CYP3A4/7 expression-inducing drug based on the expression of the two fluorescence reporter genes.

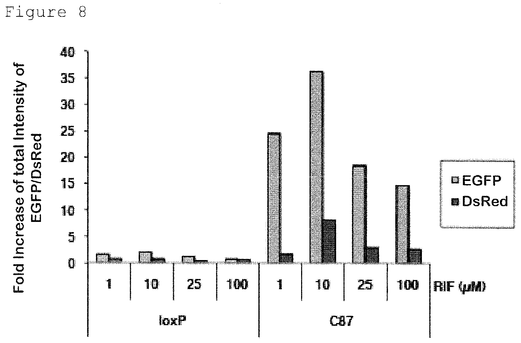

Figure 8 is a graph showing the results obtained by analyzing total area fluorescence intensity under a fluorescence microscope, in order to evaluate the inducibility of the expression of a CYP3A4G/7R BAC-introduced HepG2 cell clone (C87) 48 hours after addition of a CYP3A4/7 expression-inducing drug (RIF: rifampicin) based on the expression of the two fluorescence reporter genes. It can be confirmed that the expression of EGFP and DsRed is induced by the human CYP3A4 expression-inducing drug in the CYP3A4G/7R BAC-introduced HepG2 cell clone, compared with a control group (loxP).

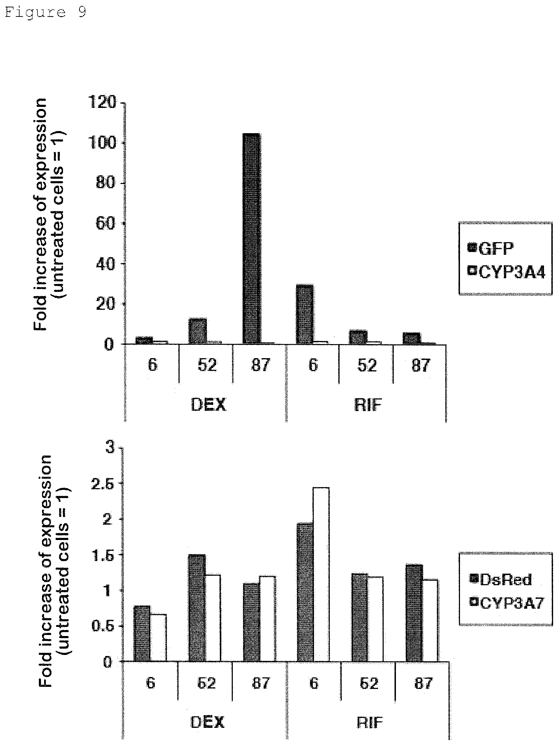

Figure 9 includes graphs showing the analysis results of quantitative RT-PCR for evaluating whether or not changes in the gene expressions of endogenous CYP3A4 and CYP3A7 genes in representative CYP3A4G/7R BAC-introduced HepG2 cell clones (C6, C52, and C87) 24 hours after addition of CYP3A4/7 expression-inducing drugs (RIF: rifampicin and DEX: dexamethasone) can be predicted based on changes in the expression levels of the fluorescence reporter genes EGFP and DsRed. In particular, these are graphs showing that the fold increase of expression of EGFP (untreated control group = 1) is higher than that of endogenous CYP3A4, and thus that evaluation of the presence or absence of the expression inducibility of the drug becomes easy.

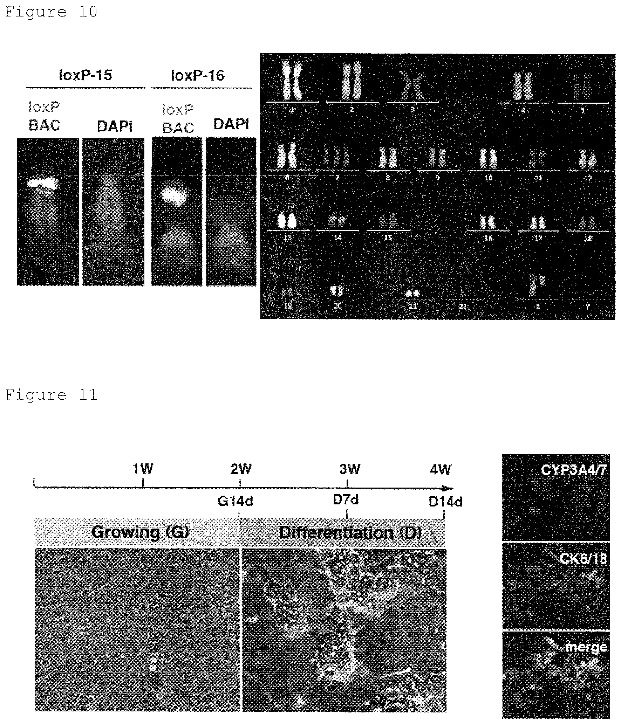

Figure 10 (left) is a FISH mapping photograph showing loxP-15 HepaRG cells in which the loxP site is introduced into human chromosome 15, and loxP-16 HepaRG cells in which the loxP site is introduced into human chromosome 16. This figure also shows that CYP3A4G/7R BAC (red) is inserted into a loxP-inserted site (green). Figure 10 (right) is an image showing the results of multicolor FISH analysis indicating a chromosomal configurations of representative CYP3A4G/7R BAC-introduced HepaRG cell clones.
Figure 11 is photographs showing confirmation of the properties of HepaRG cells. Figure 11 (left) is a photograph showing that a small hepatocyte-like cell population appeared in the upper layer of the cell population by carrying out a differentiation culture in a differentiation-dedicated medium following a growing culture in a growth-dedicated medium for 2 weeks (2W). Figure 11 (right) includes photographs of the stained antibodies showing that this hepatocyte-like cell population becomes strongly positive to an antibody recognizing both CYP3A4 and CYP3A7 and that the expression of any of them is high.

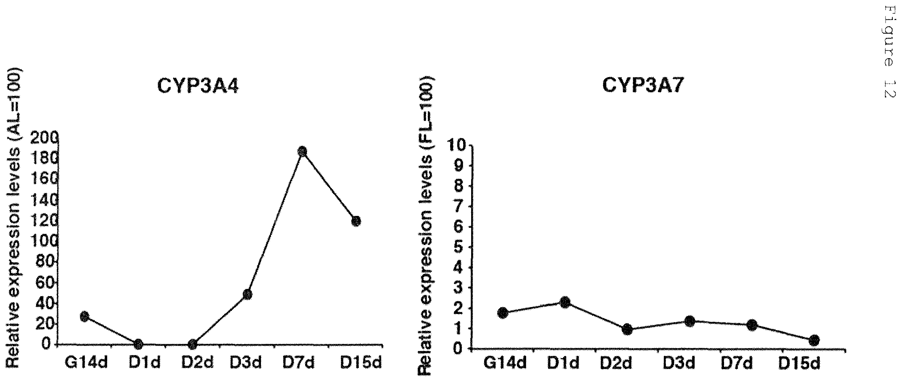

Figure 12 includes graphs showing the results obtained by analyzing, by quantitative RT-PCR, changes in the gene expressions of endogenous CYP3A4 (left) and CYP3A7 (right) in differentiation of HepaRG cells. These graphs confirms a decrease in the expression of CYP3A7 and an increase in the expression of CYP3A4, which are associated with cell differentiation, in HepaRG cells on Days 1, 2, 3, 7, and 15 (D1d, D2d, D3d, D7d, and D15d) of a differentiation culture in a differentiation-dedicated medium following a growing culture in a growth-dedicated medium for 14 days (G14d).

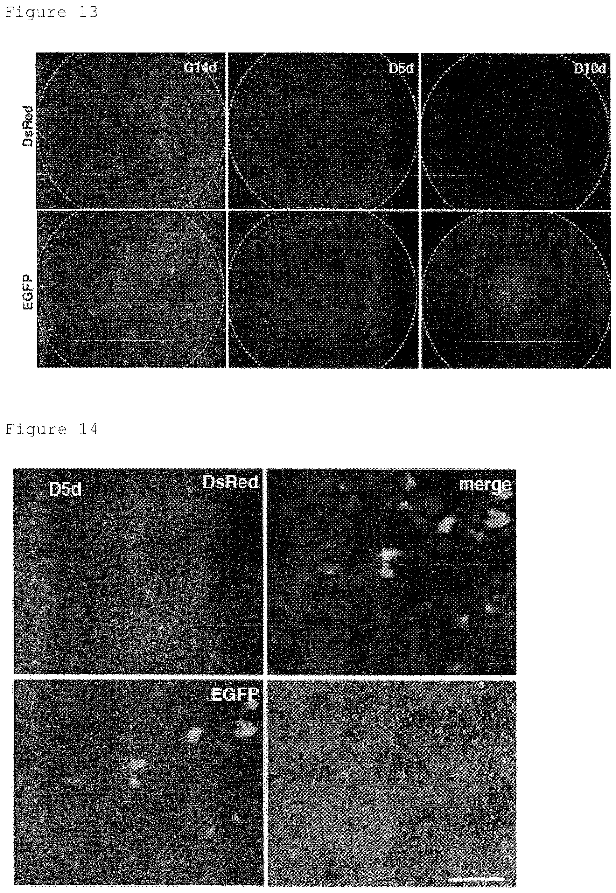

Figure 13 includes fluorescence microphotographs showing a decrease in the number of DsRed-positive cells (upper) and an increase in the amount of EGFP-positive cells (lower) after the growing culture of a representative CYP3A4G/7R BAC-introduced HepaRG cell clone (C3) in a growth-dedicated medium for 14 days (G14d), and Days 5 (D5d) and 10 (D10d) of a differentiation culture in a differentiation-dedicated medium.
Figure 14 includes microphotographs showing that CYP3A4G/7R BAC-introduced HepaRG cells have initially been all DsRed-positive cells, but that the expression of DsRed has disappeared accompanying differentiation, and some of the cells have then become EGFP-positive cells. Figure 14 shows that CYP3A4G/7R BAC-introduced HepaRG cells are used as materials for analyzing the developmental stage of CYP3A4-positive hepatocytes having high metabolic function.

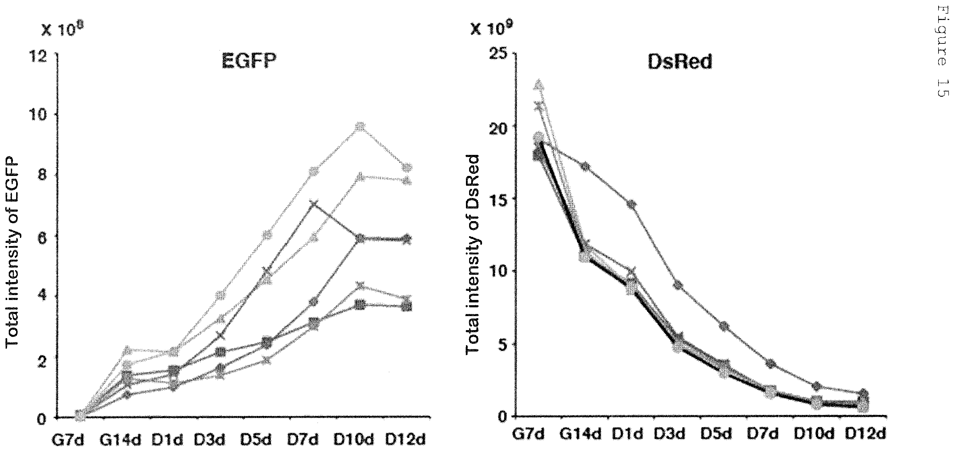

Figure 15 includes graphs showing the results obtained by analyzing, under a fluorescence microscope, a change over time in the total area fluorescence intensity of EGFP (left) and DsRed (right) on Days 7 day (G7d) and 14 (G14d) of a growing culture in a growth-dedicated medium, and Days 1, 3, 5, 7, 10, and 12 (D1d, D3d, D5d, D7d, D10d, and D12d) of a differentiation culture in a differentiation-dedicated medium. It is found that in the CYP3A4G/7R BAC-introduced HepaRG cells, a decrease in the expression of DsRed and an increase in the expression of EGFP occurred accompanying cell differentiation.

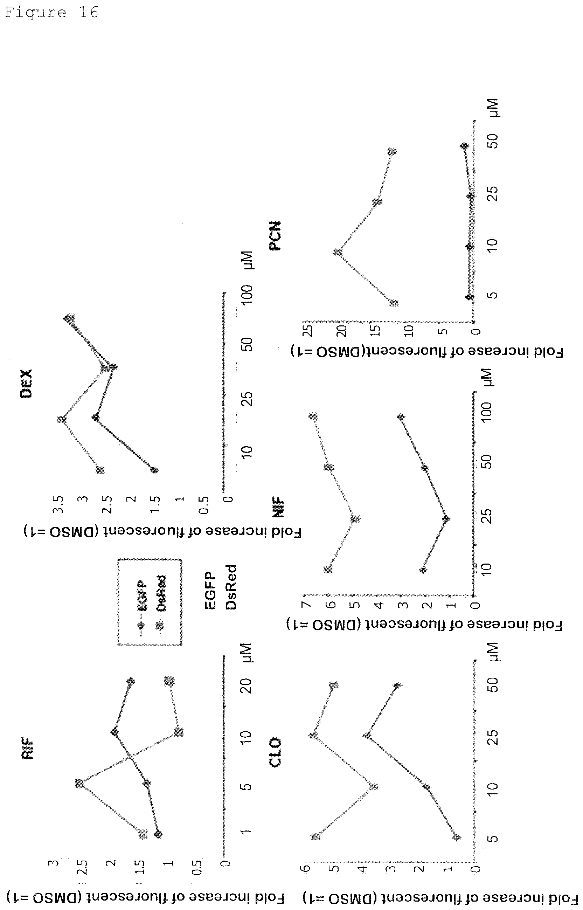

Figure 16 includes graphs showing the results obtained, by analyzing under a fluorescence microscope, the total area fluorescence intensity of EGFP and DsRed 48 hours after addition of CYP3A4/7 expression-inducing drugs (RIF: rifampicin, DEX: dexamethasone, CLO: clotrimazole, NIF: nifedipine, and PCN: pregnenolone 16α-carbonitrile) dissolved in 1% DMSO to a representative CYP3A4G/7R BAC-introduced HepaRG cell clone (C3). EGFP was shown to be induced by the drugs other than PCN. PCN has been known as a CYP-inducing drug in rodents, and it does not induce CYP3A4 in humans. Thus, non-inducibility by PCN is an example of showing that the reaction in humans can be predicted with EGFP. In addition, considering the fold increases of fluorescent of DsRed (DMSO control group = 1) by CLO, NIF and PCN that exhibited high cellular damage to HepaRG cells, these graphs show that an increase in the expression of CYP3A7 becomes a guideline for evaluation of cytotoxicity.

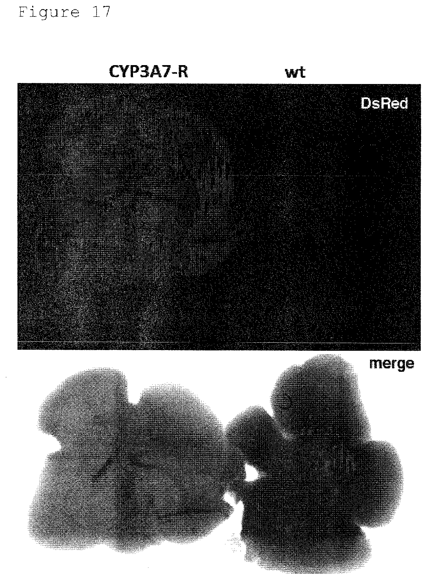

Figure 17 includes photographs showing confirmation of the expression of DsRed in the liver on the 1st day after the birth of CYP3A4+/7R MAC-introduced mice obtained by breeding chimeric mice with CYP3A4+/7R BAC-mounted MAC-introduced mouse ES cells (wt: control group).

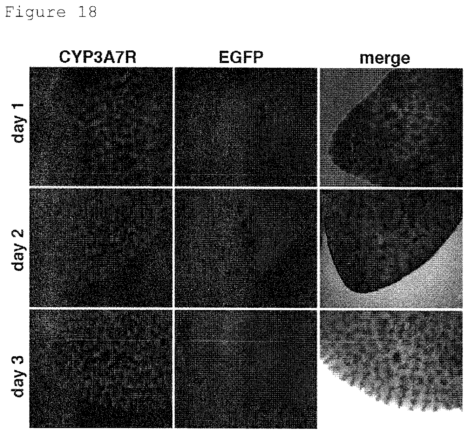

Figure 18 includes fluorescence microphotographs of the liver of an adult CYP3A4+/7R MAC-introduced mouse, to which 5% carbon tetrachloride was administered as a liver-damaging substance in order to study whether liver injury can be evaluated by an increase in the expression of DsRed. From the photographs, it is found that the suppressed fluorescence intensity of DsRed in the liver increases after the mouse has grown up. In the CYP3A4+/7R MAC-introduced mouse, EGFP functions as a marker showing introduction of MAC into cells.

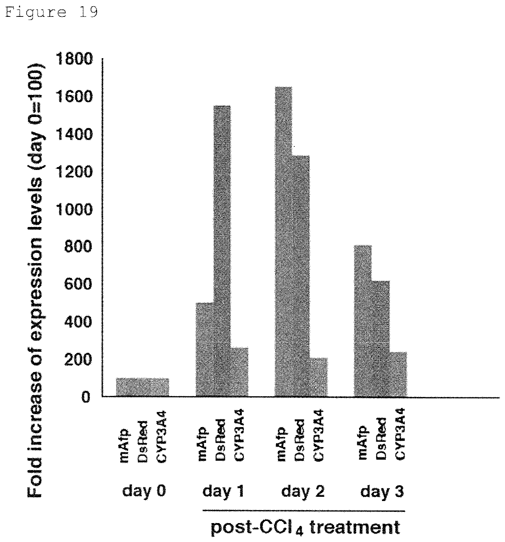

Figure 19 is a graph showing the results of quantitative RT-PCR analysis demonstrating that the suppressed expression of DsRed in the liver of an CYP3A4+/7R MAC-introduced mouse increases after the mouse has grown up, if 5% carbon tetrachloride was administered as a liver-damaging substance to the adult mouse, in order to study whether liver injury can be evaluated based on an increase in the expression of DsRed in the CYP3A4+/7R MAC-introduced mouse. It is found that DsRed can be used as a liver regeneration marker after hepatotoxicity, as well as an α-fetoprotein (Afp) that is a marker for immature hepatoblasts.

### Description of Embodiments

The present specification discloses a vector. Examples of the vector include a plasmid vector, a cosmid vector, a viral vector, and an artificial chromosome vector. Examples of the artificial chromosome vector include a yeast artificial chromosome vector (YAC), a bacterial artificial chromosome vector (BAC), a p1 artificial chromosome vector (PAC), a mouse artificial chromosome vector (MAC), and a human artificial chromosome vector (HAC).

The vector disclosed herein comprises the expression control region of a fetus-specific gene.

The fetus-specific gene is a gene whose expression specifically increases during a fetal period. As such fetus-specific genes, various types of genes have been known. Examples of the fetus-specific gene include CYP3A7, Cyp3a16, α-fetoprotein, γ-glutamyl transpeptidase, GST-P, M2-PK, CK8, CK18, RL16/79, and EpCAM. CYP3A7 (cytochrome P450, family 3, subfamily A, polypeptide 7) is explained as Registration No. NM 000765 in NCBI Resources. The sequence of CYP3A7 is shown in Registration No. NM000765.3. CYP3A7 is a drug-metabolizing enzyme gene as well as a fetus-specific gene. Thus, CYP3A7 can be used as an indicator in evaluation of the induction of a drug-metabolizing enzyme, and thereby, it becomes possible to carry out more accurate evaluation. CYP3A7 is a marker for immature hepatoblasts. Using CYP3A7, liver regeneration after hepatocyte toxicity can be evaluated.

The expression control region is a region that controls the expression of a gene located downstream thereof. Examples of such an expression control region include a promoter and an enhancer. The promoter is a DNA sequence associated with initiation of the transcription of the coding region of a gene to which it binds. The enhancer controls the specificity of the bound promoter. The expression control region may comprise either a single promoter or a single enhancer, but it may also comprise both of the promoter and the enhancer.

The expression control region of a fetus-specific gene can be obtained by a well-known technique based on known information such as GenBank. Regarding CYP3A7, for example, Registration No. AF181861.1 of GenBank discloses a 1012-bp nucleotide sequence as a human cytochrome P-450IIA7 (CYP3A7) gene, a promoter region and a partial sequence. This 1012-bp nucleotide sequence is shown in SEQ ID NO: 1. Moreover, GenBank also discloses Registration No. AC069294.5 that is the nucleotide sequence of a BAC clone RP11-757A13 comprising the expression control region of CYP3A7. The nucleotide sequence disclosed as Registration No. AC069294.5 is shown in SEQ ID NO: 2. Furthermore, since the expression control region of CYP3A7 shows homology of 90% or more with the expression control region of CYP3A4, the promoter and enhancer regions of CYP3A7 are known to be in relative positions to the promoter and enhancer regions of CYP3A4 with Registration No. AF185589.1 (Bertilsson, G. et al., BBRC 280, 139-144, 2001; Sueyoshi, T and Neishi, M., Annu. Rev. Pharmacol. Toxicol. 41: 123-43, 2001). A sequence ranging from the transcription start point to the point at approximately - 8000 bp of CYP3A7 can be used as an expression control region of CYP3A7. Sequences ranging from the transcription start point to -7733 to -7719 (dNR1), -7689 to -7672 (dNR2), -7287 to -7273 (dNR3), and -171 to -154 (pNR) of Registration No. AF185589.1 are known to be enhancers of CYP3A4. Four homologous regions in CYP3A7 that show homology with these sequences are also known to function as enhancers. The sequences of these 4 enhancers are shown in the following Table 1. The expression control region of CYP3A7 preferably comprises the aforementioned 4 enhancer regions. Using the sequence ranging from the transcription start point to the point at approximately -8000 bp as an expression control region of CYP3A7, the expression control region of CYP3A7 can comprise these enhancers. In addition, sequences having identity of 95%, 98%, or 99% or more with these sequences can also be used as expression control regions of CYP3A7, as long as they have a function to control the expression of a gene located downstream thereof. Herein, the identity of sequence can be calculated using analysis tools that are commercially available or are made available through electric telecommunication lines. Specifically, such sequence identity can be calculated using analysis software such as BLAST (J. Mol. Biol., 215, 403, 1990) or FASTA (Methods in Enzymology, 183, 63-69).

**[Table 1]**

| dNR1 -7733∼-7719 [a part of XREM] | |
|---|---|
| TGAACTtgcTGACCC | CYP3A4 |
| TGAACTtgcTGACCC | CYP3A7 |

| dNR2 -7689∼-7672 [a part of XREM] | |
|---|---|
| TGAAATcatgtcGGTTCA | CYP3A4 |
| TGAAATcatgtcAGTTCA | CYP3A7 |

| dNR3 -7287∼-7273 | |
|---|---|
| TATTGTtatTGAACT | CYP3A4 |
| TATTATtatTGAACT | CYP3A7 |

| pNR -171∼-154 [alias: PrPXRE] | |
|---|---|
| TGAACTcaaaggAGGTCA | CYP3A4 |
| TTAACTcaatggAGGTCA | CYP3A7 |

In the table, the capital letter indicates a nuclear receptor sequence, the small letter indicates a spacer sequence, and the underline indicates a different site between the two genes.

The vector disclosed herein comprises a first reporter gene.

The reporter gene is any given nucleic acid sequence that allows a cell to present a detectable label. Examples of such a detectable label include a fluorescence signal, a phosphorescence signal, a protein that is detectable in an assay, an enzyme activity, and an antigen that is detectable on a cell or in a cell. Examples of a protein encoded by such a reporter gene include fluorescent proteins such as a green fluorescent protein (GFP), a humanized Renilla green fluorescent protein (hrGFP), an enhanced green fluorescent protein (eGFP), an enhanced blue fluorescent protein (eBFP), an enhanced cyan fluorescent protein (eCFP), an enhanced yellow fluorescent protein (eYFP), and a red fluorescent protein (RFP or DsRed). More examples of a protein encoded by such a reporter gene include bioluminescent proteins such as firefly luciferase and Renilla luciferase. Further examples of a protein encoded by such a reporter gene include enzymes for converting chemiluminescent substrates, such as alkaline phosphatase, peroxidase, chloramphenicol acetyltransferase, and β-galactosidase. In the present invention, when a reporter gene detected by a light signal such as a fluorescence signal or a phosphorescence signal is used, the expression level of the reporter gene can be observed in a state in which a cell is allowed to survive, and a cell used for evaluation can be easily selected, while the cell is alive. In addition, in such a case, the reporter gene can be used in an experiment in which a test substance is continuously administered, and a change over time in the expression level of the reporter gene can be pursued in a real time. As such, a reporter gene using a light signal as a label can be preferably used as the reporter gene of the present invention.

The vector disclosed herein comprises the expression control region of a drug-metabolizing enzyme gene.

The drug-metabolizing enzyme gene is a gene that encodes an enzyme metabolizing a drug in a living body. Various genes have been known as such drug-metabolizing enzyme genes. Examples of the known drug-metabolizing enzyme gene include proteins binding to cells or being present on cells and nuclear proteins, which chemically modify or isolate drugs. Specific functions of the drug-metabolizing enzyme gene, such as chemical decomposition, conversion of a drug to another compound, or transportation of a drug between other cells or in an intracellular space, are not particularly limited, as long as the drug-metabolizing enzyme gene acts to eliminate a drug from a living body or to substantially change the effects of the drug. Examples of the drug-metabolizing enzyme gene include cytochrome P450, flavin-containing monooxygenase, alcohol dehydrogenase, aldehyde dehydrogenase, monoamine oxidase, aldehyde reductase, ketone reductase, esterase, epoxy hydrolase, β-glucuronidase, sulfatase, UDP-glucuronic acid transferase, glutathione S-transferase, N-acetyltransferase, sulfotransferase, glycine conjugation enzyme, methylase, and glucose transferase. Cytochrome P450 includes a CYP3A gene group including CYP3A4, CYP3A7 and the like (which is also simply referred to as "CYP3A"). Moreover, such cytochrome P450 includes CYP1A1, CYP1A2, CYP1B1, CYP2A6, CYP2A13, CYP2B6, CYP2C8, CYP2C9, CYP2C19, CYP2D6, CYP2E1, CYP2J2, CYP3A4, CYP3A5, CYP3A7, CYP4B1, CYP5A1, CYP8A1, CYP21, and the like. The gene having a role in transportation include OCT1, NTCP, OATP1B1, OATP1B3, OATP2B1, OAT2, MRP2, MRP3, MRP4, MRP6, MDR1, BCRP, BSEP, and the like. The nuclear receptor includes PXR, CAR, and the like.

The expression control region of a drug-metabolizing enzyme gene can be obtained by a well-known technique, based on known information such as GenBank. For example, Registration No. AF185589.1 of GenBank discloses a 11374-bp sequence as a promoter region of a human cytochrome P450 3A4 (CYP3A4) gene. A sequence ranging from the transcription start point to the point at approximately - 8000 bp can be used as an expression control region of CYP3A4. This 11374-bp sequence disclosed as Registration No. AF185589.1 is shown in SEQ ID NO: 3. In particular, sequences ranging from the transcription start point to - 7733 to -7719 (dNR1), -7689 to -7672 (dNR2), -7287 to - 7273 (dNR3), and -171 to -154 (pNR) of Registration No. AF185589.1 are known to be enhancers of CYP3A4 (Bertilsson, G. et al., BBRC 280, 139-144, 2001; Sueyoshi, T and Neishi, M., Annu. Rev. Pharmacol. Toxicol. 41: 123-43, 2001). These sequences may be used as expression control regions of CYP3A4. In addition, sequences having identity of 95%, 98%, or 99% or more with these sequences can also be used as expression control regions of CYP3A4, as long as they have a function to control the expression of a gene located downstream thereof. Herein, the identity of sequence can be calculated using analysis tools that are commercially available or are made available through electric telecommunication lines. Specifically, such sequence identity can be calculated using analysis software such as BLAST (J. Mol. Biol., 215, 403, 1990) or FASTA (Methods in Enzymology, 183, 63-69). When the expression control region of CYP3A4 is used, high expression of a second reporter gene located downstream thereof becomes an indicator showing that the concerned cells are suitable for evaluation of induction of a drug-metabolizing enzyme. Thus, it becomes possible to prepare cells used for more accurate evaluation.

The vector disclosed herein comprises a second reporter gene. The second reporter gene is desirably different from the first reporter gene.

In the vector of the present invention, the first reporter gene is located downstream of the expression control region of an early developmental stage-specific gene, and the second reporter gene is located downstream of the expression control region of a drug-metabolizing enzyme gene. If the reporter gene is positioned after the expression control region when a direction in which the reporter gene is transcribed is used as a reference, it may be determined that the reporter gene is located downstream of the expression control region. Also, when the expression control region is operably linked to the reporter gene, it may be determined that the reporter gene is located downstream of the expression control region. When experimental results are obtained in which the expression control region drives the transcription of a coding region, it may be considered that the expression control region operably binds to the coding region.

The vector of the present invention may comprise a sequence used for genetic recombination of utilizing a site-specific recombination reaction such as a Cre-loxP system. When the vector of the present invention comprises a sequence used for the Cre-loxP system, it becomes possible to introduce one copy of the entire-length vector into all cells. Thereby, a variation in the expression of a reporter gene among cells is reduced, and thus, accuracy in the evaluation is further improved.

The vector of the present invention may be obtained by ligating a nucleic acid fragment containing an expression control region, a reporter gene and the like to a vector according to a known method. In addition, the present vector may also be obtained by replacing the protein coding region (ORF) of each gene in a vector comprising the peripheral region on the genome of the genes with a reporter gene.

In the vector of the present invention, when CYP3A4 is used as a drug-metabolizing enzyme gene and CYP3A7 is used as a fetus-specific gene, the expression of the two genes is under developmental stage-specific control. Accordingly, in cells that are not suitable for evaluation of induction of a drug-metabolizing enzyme, an increase in the expression of a first reporter gene and a decrease in the expression of a second reporter gene are observed. On the other hand, in cells that are suitable for such evaluation, a decrease in the expression of a first reporter gene and an increase in the expression of a second reporter gene are observed. As such, whether or not cells are suitable for evaluation of induction of a drug-metabolizing enzyme can be determined from both sides, namely, an increase in the expression of a reporter gene and a decrease in the expression thereof. Thus, functional cells, which have high maturity of differentiation and sufficiently maintain *in vivo* functions, can be extremely efficiently obtained. Accordingly, in the vector of the present invention, CYP3A4 is used as a drug-metabolizing enzyme gene and CYP3A7 as a fetus-specific gene.

The vector of the present invention can be used for production of cells used to evaluate the induction of a drug-metabolizing enzyme by a test substance.

The cells used to evaluate the induction of a drug-metabolizing enzyme by a test substance can be obtained by introducing the vector of the present invention into cells according to a known method such as lipofection or a microcell fusion method.

The cells into which the vector of the present invention is to be introduced are cells in tissues in a living body, in which induction of a drug-metabolizing enzyme occurs. Examples of the tissues, from which the cells into which the vector of the present invention is to be introduced are derived, include liver and small intestine. The organism, from which the cells into which the vector of the present invention is to be introduced are derived, is desirably an organism in which induction test of a drug-metabolizing enzyme is required. Examples of such an organism include mammals such as a human and a mouse. Moreover, the cells into which the vector of the present invention is to be introduced may be cultured cells, such as HepG2 cells, HepaRG cells, and Hu7 cells. The HepG2 cells are human hepatoma cells, which have been widely used as human hepatocyte model cells. The cells into which the vector of the present invention is to be introduced are preferably hepatoma-derived cell lines, such as HepaRG (registered trademark). Moreover, the cells into which the vector of the present invention is to be introduced may be pluripotent stem cells, such as embryonic stem cells (ES cells), and induced pluripotent stem cells (iPS cells). Such embryonic stem cells and induced pluripotent stem cells are able to grow indefinitely, and thus, these cells are useful as a large supply source of functional cells. The cells into which the vector of the present invention is to be introduced are preferably immature cells having ability to differentiate into hepatocytes having high maturity, such as hepatoma-derived cell lines having the properties of stem cells capable of differentiating into mature hepatocytes; hepatoma-derived cell lines or stem cells in the liver in a living body; and ES cells or iPS cells.

The cells used to evaluate the induction of a drug-metabolizing enzyme by a test substance may also be cells obtained by inducing pluripotent stem cells, into which the vector of the present invention has been introduced, to differentiate into preferred tissues. For example, in the case of cells exhibiting the properties of the mature hepatocytes of an adult as their functions, the cells are induced to differentiate into hepatocytes by a method comprising obtaining SOX9-positive progenitor cells that are common in the pancreas, the liver and the small intestine from pluripotent stem cells such as iPS cells, into which the vector has been introduced, then obtaining AFP/PDX1-positive progenitor cells that are common in the pancreas and the liver, and then allowing the obtained cells to grow, and thereafter, CYP3A7/Afp/EpCAM-positive fetal hepatocytes are obtained. Then, maturation of the cells is carried out to obtain CYP3A4/ALB-positive adult liver, so that the cells of interest may be obtained.

Moreover, as such cells used to evaluate the induction of a drug-metabolizing enzyme by a test substance, a chimeric animal may be produced using pluripotent stem cells into which an artificial chromosome has been introduced according to a microcell fusion method or the like, and thereafter, cells may be fractionated from tissues in the liver or the like of the produced chimeric animal and may be then used. By selecting cells having low expression of the first reporter gene from the fractionated cells, cells capable of more accurately evaluating the induction of a drug-metabolizing enzyme can be obtained. Examples of the non-human animal comprising the vector of the present invention include mammals such as bovine, miniature pigs, pigs, sheep, goats, rabbits, dogs, cats, guinea pigs, hamsters, mice, rats, and monkeys.

Cells, which comprises the vector of the present invention used to produce cells used for evaluation of the induction of a drug-metabolizing enzyme by a test substance and the cytotoxicity of the test substance, and which involves a low expression level of the gene by the expression control region of a fetus-specific gene in the vector of the present invention, have high maturity and are suitable for tests for evaluating the induction of a drug-metabolizing enzyme. Accordingly, the cell used to evaluate the induction of a drug-metabolizing enzyme by a test substance is preferably a cell selected by a method comprising: (1) a step of culturing cells containing the vector of the present invention; and (2) a step of selecting a cell, using the expression level of the first reporter gene in the cells during the culture in the step (1) as an indicator. More specifically, in step (2), a cell in which the expression level of the first reporter gene is low is selected. Among the above described cells, cells, in which the expression level of the second reporter gene by the expression control region of a drug-metabolizing enzyme gene in the vector of the present invention is high, are have higher maturity, and thus, such cells are more suitable for tests for evaluating induction of a drug-metabolizing enzyme. Accordingly, more preferably, in step (2), cells are selected, using the expression level of the second reporter gene in cells as an indicator. More specifically, in step (2), cells, in which the expression level of the second reporter gene is high, are preferably selected. Moreover, the culture in step (1) is preferably carried out to allow cells containing the vector of the present invention to differentiate into hepatocytes. The culture that is carried out to allow the cells to differentiate into hepatocytes can be carried out in a medium used for differentiation into hepatocytes. The cells containing the vector of the present invention, which are cultured in step (1), may be selected, using the expression of the first reporter gene as an indicator. Furthermore, cells may be selected in step (2), using the presence of the cells in the upper layer of the cell population as a further indicator.

The thus obtained cells can be used to evaluate the induction of a drug-metabolizing enzyme by a test substance. The test substance is a product used to verify if it causes induction of a drug-metabolizing enzyme, and an example of the test substance is a pharmaceutical product candidate compound. Examples of the test substance include, but are not limited to, pharmaceutical products, food products, chemical substances, and the metabolites thereof.

The evaluation method disclosed herein comprises a step of evaluating the expression of the first reporter gene in the above-obtained cells used to evaluate the induction of a drug-metabolizing enzyme by a test substance (hereinafter simply referred to as "cells for evaluation"). More specifically, the evaluation method disclosed herein may comprise a step of evaluating the expression of the first reporter gene in the cells and then removing cells involving high expression of the first reporter gene from the cells used for evaluation. Moreover, the evaluation method disclosed herein may comprise a step of allowing a test substance to come into contact with a cell for evaluation, evaluating a change in the expression of the first reporter gene between before and after the contact, and determining that the contacted test substance has cytotoxicity when the increase in the expression is observed. The contact of the test substance with the cell for evaluation may be carried out by administering the test substance to a non-human animal comprising the vector disclosed herein.

The evaluation method disclosed herein comprises a step of evaluating a change in the expression of the second reporter gene in the cell for evaluation between before and after the contact of the cell for evaluation with the test substance. More specifically, the evaluation method disclosed herein may comprise a step of determining that the contacted test substance has ability to induce a drug-metabolizing enzyme, when the expression of the second reporter gene is increased by the contact with the test substance. Preferably, a change in the expression of the second reporter gene is evaluated based on continuous measurement results of the expression of the second reporter gene. In this case, the use of the continuous measurement results is preferable in that, even in a case where the expression of the second reporter gene in the cell is decreased after it has been once increased by drug enzyme induction, the drug enzyme induction can be carried out simply and accurately, without missing the results. Such a continuous measurement is carried out, for example, for 3 days after the contact with the test substance. The continuous measurement is carried out by obtaining the results of the expression level of the reporter gene, for example, every one or two hours, and preferably every 30 minutes to 1 hour.

The evaluation method provided by the present invention may be carried out by allowing a plurality of test substances to simultaneously come into contact with cells using a plurality of wells, such as 96 wells or 384 wells, so that the present evaluation method may be carried out by high content screening (HCS) for simultaneous evaluation of reporter genes. When a reporter gene using a light signal as a label is used, the expression of the reporter gene can be simply discriminated as light signal at a time, and thus, a large number of test substances can be promptly evaluated.

The evaluation method provided by the present invention may be carried out using a kit. An example of such a kit is a kit for evaluating the induction of a drug-metabolizing enzyme by a test substance and the cytotoxicity of the test substance, wherein the kit comprises the vector of the present invention. The kit of the present invention may further comprise: cells; a medium for culturing the cell line and allowing the cells to grow; a drug for inducing differentiation of the cells into hepatocytes; a test substance; a vessel for culturing the cells; an instruction manual regarding at least one constituent of the kit; and the like. The cells comprised in the kit are preferably cells having the properties of stem cells capable of differentiating into mature hepatocytes.

### [Examples]

Hereinafter, the present invention will be described in the following examples. However, these examples are not intended to limit the scope of the present invention.

### Example 1 Use of bacterial artificial chromosome (BAC) :

### (1) Production of bacterial artificial chromosome comprising protein coding region (ORF) of CYP3A4 and CYP3A7:

A bacterial artificial chromosome RP11-757A13 BAC comprising the gene expression control regions and protein coding regions of CYP3A4 and CYP3A7, which was produced by inserting the gene expression control region ranging from CYP3A4 to CYP3A7 (an entire length of 123,778 bp) into the EcoRI site of a 11.6-kb BAC clone vector pBACe3.6, was used in the subsequent experiment. The nucleotide sequence of the BAC clone RP11-757A13 is disclosed as Registration No. AC069294.5 in GenBank.

### (2) Substitution of protein coding regions of CYP3A4 and CYP3A7 into EGFP and DsRed:

### a. Production of vector used for substitution into DsRed:

Using a pDsRed-Express-1 vector as a basic vector, the 5' region (left arm, LA) of hCYP3A7 was inserted into the BglII-SalI site located immediately before the ORF of a DsRed gene, and the 3' region (right arm, RA) of CYP3A7 was inserted into the BsaI site located immediately after a kanamycin-resistant gene, so as to construct an LA-DsRed-pA-RA CYP3A7 knock-in (hereinafter also referred to as simply "KI") vector that is a vector used for substitution into DsRed.

### b. Production of vector used for substitution into EGFP

Using a pHygEGFP vector as a basic vector, the LA and RA of CYP3A4 were inserted into the BglII site comprising pA and an ampicillin-resistant gene, such that after open chain, the EGFP gene can be located between LA and RA, so as to construct an LA-EGFP-pA-RA-CYP3A4 KI vector that is a vector used for substitution into EGFP.

### c. Substitution into DsRed

### (a) Introduction of bacterial artificial chromosome into cells used for homologous recombination

DY380 was streaked onto an LB plate, and it was then heated at 32°C overnight. A liquid prepared by picking up colonies and subjecting them to a shaking culture (32°C, 180-200 rpm, 14 hours) was transferred into a 50-ml LB liquid, and it was then subjected to a shaking culture (32°C, 180-200 rpm, 3 to 4 hours). When the O.D. became 0.4, shaking was terminated, and the cell culture solution was then stirred in a water bath at 42°C for 5 minutes. After that, the cell culture solution was left at rest for 5 minutes. Then, after it had been left at rest in ice for 10 minutes, DY380 was collected using a centrifuge (4°C, 3500 rpm, 10 minutes). After the collected cells were washed with MQ twice, they were then mixed with 200 ng of the aforementioned RB11-757A13 BAC DNA, followed by performing electroporation with Gene Pluser (1750 V, 25 µF, 200 Ω, pulse no. 1). The resultant was shaken at 32°C for 1.5 hours, and thereafter, it was applied onto an LB plate and was then heated at 32°C overnight. In this way, the bacterial artificial chromosome RB11-757A13 was introduced into the DY380 cells that were cells used for homologous recombination, so as to obtain a (CYP3A4+/7+) BAC clone comprising both CYP3A4 and CYP3A7.

### (b) Substitution into DsRed by homologous recombination

A necessary DNA fragment was cleaved from the aforementioned LA-DsRed-pA-RA CYP3A7 KI vector that was a vector used for substitution into DsRed, and 100 ng of the DNA fragment was introduced into the aforementioned CYP3A4+/7+ BAC clone by electroporation. It was applied onto a drug-added LB plate. Genomic PCR was performed on the obtained kanamycin-resistant clone, using primers for confirmation of homologous recombination. The used primers are shown in Table 2. From all BAC clones, PCR products having the estimated length were obtained. Thus, a CYP3A4+/7R BAC clone, in which the ORF of CYP3A7 was substituted with DsRed, was obtained.

### [Table 2-1]

**Table 2. Genomic PCR primers for confirmation of CYP3A4/7BAC and KI and RT-PCR primers for confirmation of gene expression**

| SEQ ID NO: | Primer name | Nucleotide sequence | Remarks |
|---|---|---|---|
| 4 | CYP3A4-1F | | |
| 5 | CYP3A4-1R | | |
| 6 | CYP3A4-2F | | |
| 7 | CYP3A4-2R | | |
| 8 | CYP3A4-3F | | |
| 9 | CYP3A4-3R | TTGCAAGCCACCCCTCTGCG | |
| 10 | CYP3A4-4F | | |
| 11 | CYP3A4-4R | | |
| 12 | CYP3A7-1F | | |
| 13 | CYP3A7-1R | | |
| 14 | CYP3A7-2F | | |
| 15 | CYP3A7-2R | | |
| 16 | CYP3A7-3F | | |
| 17 | CYP3A7-3R | | |
| 18 | CYP3A7-4F | | |
| 19 | CYP3A7-4R | | |
| 20 | 5' EGFP | CAGCTCCTCGCCCTTGCTCAC | R for confirmation of EGFP vector LA recombination |
| 21 | Amp^rf | GTAGAAAAGATCAAAGGATC | F for confirmation of EGFP vector RA recombination |
| 22 | EGFP_pA_F_ | | EGFP gene confirmation |

**[Table 2-2]**

| SEQ ID NO: | Primer name | Nucleotide sequence | Remarks |
|---|---|---|---|
| 23 | EGFP_pA_R_ | GCCTCTTCGCTATTACGCCAG | |
| 24 | 5' DsRed-R | GATGACGTCCTCGGAGGAGGC | R for confirmation of DsRed vector LA recombination |
| 25 | Kan/Neo^ R-F | GCCTTCTTGACGAGTTCTTC | F for confirmation of DsRed vector RA recombination |
| 26 | DsRed-F | GCCTCCTCCGAGGACGTCATC | Confirmation of DsRed gene |
| 27 | Kan/Neo-R | | |
| 28 | N-EGFP-R | CGCCGTCCAGCTCGACCAGGAT | EGFP vector LA recombination |
| 29 | N-Amp-F | CTGAGATAGGTGCCTCACTGA | F for confirmation of EGFP vector RA recombination |
| 30 | 500bpRA-F | | Production of EGFP vector 500 bp RA |
| 31 | 500bpRA-R | GCCAACAGTGATTACAATGACC | |
| 32 | LacZ770As | | Confirmation of loxP vector LA recombination |
| 33 | BAC e3.64168S | | |
| 34 | SLR 1650S | | Confirmation of loxP vector RA recombination |
| 35 | BAC e3.65839AS | | |

### d. Substitution into EGFP:

The above described LA-EGFP-pA-RA-CYP3A4 KI vector was linearized with xhoI, and the obtained linear DNA was knocked in the CYP3A4 ORF region on the above-obtained CYP3A4+/7R BAC by electroporation, so that the EGFP gene was inserted therein. Using 9 pairs of primers for confirming homologous recombination, genomic PCR was carried out on the obtained ampicillin-resistant clone. As a result, it was confirmed that PCR products having the estimated length were found in all of the obtained clones. In this way, a CYP3A4G/7R BAC clone that was a BAC clone in which the ORF of CYP3A7 was substituted with DsRed and the ORF of CYP3A4 was substituted with EGFP was obtained. The primers used in the genomic PCR are shown in the above Table 2.

### e. Insertion of a loxP site:

In order to mount BAC DNA via a Cre/loxP system onto MAC (mouse artificial chromosome) or any given chromosome into which a loxP sequence had previously been inserted, a loxP sequence was knocked in the above-obtained CYP3A4G/7R BAC according to a known method (Sternberg and Hamilton, 1981; Hoess et al., 1982; Nagy, 2000).

### Example 2 Production of cells into which bacterial artificial chromosome is introduced:

### (1) Production of loxP tg-HepG2:

A plasmid vector (VH21-12#8-3) comprising HPRT exon 1-2, loxP, and a Hyg-resistant gene as a drug selection marker was treated with the restriction enzyme (NotI) to linearize it. Lipofectamine LTX reagent was used for introduction of this vector into HepG2. First, 5 µg of VH21-12#8-3 was diluted with 2.5 ml of Opti-MEM, and 12.5 µg of PLUS reagent was added to the diluted solution. The obtained mixture was left at rest at room temperature for 5 minutes. Thereafter, 68.75 µl of LTX reagent was added to the reaction solution, and the obtained mixture was left at rest at room temperature for 25 minutes. Thereafter, the reaction solution was added onto HepG2 (10-cm dish, approximately 1 × 10⁷). It was cultured in 5 ml of D-MEM that contained 10% fetal bovine serum for 1 day, and HepG2 was removed from the culture dish by treating it with trypsin, and it was then seeded on five 10-cm dishes. Hygromycin (400 µg/ml) was added to a medium, and the medium was then exchanged with a fresh one every 3 days. On Day 14 after addition of hygromycin, colonies were isolated, and loxP tg-HepG2, a transgenic HepG2 cell line having one loxP site on the long arm of chromosome 14 was obtained. Gene transfer was confirmed by PCR using a primer set that amplified a region ranging from the HPRT exon 2 terminus to a hygromycin region. Upon production, when HepG2 was cultured, D-MEM (High-glucose) that contained L-glutamine and phenol red, to which 10% fetal bovine serum was added, was used. In addition, upon subculture, cells were removed using 0.04% EDTA-added 0.1% trypsin solution.

### (2) Introduction of CYP3A4G/7R BAC into loxP tg-HepG2:

In order to insert the above described CYP3A4G/7R-BAC into the above described loxP TG-HepG2 using a Cre-loxP system, lipofection was carried out using Lipofectamine LTX reagent. First, 5 µg of CYP3A4G/7R-BAC and 2 µg of pCAG-Cre were diluted with 1.25 ml of Opti-MEM, and thereafter, 6.25 µl of PLUS reagent was added to the diluted solution. The obtained mixture was left at rest at room temperature for 5 minutes. Thereafter, 25 µl of LTX reagent was added to the reaction solution, and the obtained mixture was left at rest for 25 minutes. Then, the obtained reaction solution was added onto HepG2 (6-cm dish, approximately 80% confluent). After completion of a culture for 1 day, the cells were removed from the dish by treating them with trypsin, and were then seeded on three 10-cm culture dishes. Neomycin (800 µg/ml G418) was added to a medium, and the medium was then exchanged with a fresh one every 3 days. On Day 10 after addition of neomycin, colonies were isolated, and CYP3A4G/7R BAC-introduced HepG2 cells that were a cell line prepared by introducing CYP3A4G/7R BAC into loxP tg-HepG2 were obtained. The obtainment of a stable expression cell line was confirmed by PCR using the primer sets for amplifying the EGFP and DsRed regions of a BAC vector, as shown in Table 2, and FISH analysis (Figure 1; a basic portion of the long arm of human chromosome 14; arrow).

### Example 3 Production of CYP3A4+/7R-mounted and CYP3A4G/7R-mounted mouse artificial chromosomes:

The above described CYP3A4+/7R-loxP BAC was purified, and it was then transfected together with a Cre enzyme expression vector into CHO cells retaining MAC by a lipofection method. DsRed, EGFP, and the control regions of CYP3A4 and CYP3A7 were confirmed by genomic PCR. Further, FISH analysis was carried out, and CYP3A4+/7R MAC was obtained as a clone capable of confirming MAC in the CHO cells and CYP3A4+/7R BAC mounted on the MAC (Figure 2, red, MAC; green, BAC). Likewise, CYP3A4G/7R-loxP BAC was introduced into MAC by a Cre enzyme to obtain a CYP3A4G/7R MAC clone. As such MAC, MAC having no EGFP reporters was used for the mounting of CYP3A4G/7R BAC. On the other hand, MAC in which an EGFP reporter is inserted upstream of a loxP site was used for the mounting of CYP3A4+/7R BAC.

### Example 4 Production of ES cells, into which CYP3A4+/7R mouse artificial chromosome is introduced:

The CYP3A4+/7R MAC in the CHO cells obtained in Example 3 was introduced into mouse ES cells (TT2F) by a microcell fusion method. Using the primers shown in Table 2, DsRed, EGFP, and the control regions of CYP3A4 and CYP3A7 were confirmed by genomic PCR. Moreover, FISH analysis was carried out, so that the BAC DNA mounted on the MAC in the TT2F cells could be confirmed (Figure 3-2). As such, CYP3A4+/7R ES cells were obtained. This CYP3A4+/7R ES cell clone exhibited mouse female normal karyotype 39, X0, and +MAC, pursuant to the karyotype (39, X0) of TT2F (Figure 3-1). In addition, these ES cells emitted green fluorescence as a result of the expression of EGFP on the MAC (Figure 4). Mouse A9 cells, into which the CYP3A4G/7R MAC in the CHO cells obtained in Example 3 were introduced, were also produced by the same method as that described above. A9 cells can be provided as MAC vector-providing cells, which will be more suitable for a microcell fusion method than CHO cells.

### Example 5 Obtainment of mice produced from ES cells containing CYP3A4+/7R-mounted artificial chromosome:

### (1) Production of chimeric mice from CYP3A4+/7R ES cells:

Highly chimeric baby mice were obtained using the CYP3A4+/7R mouse ES cells obtained in Example 4. All tissues, such as liver, skin, brain, lung, small intestine, spleen, and pancreas, of individual chimeric mice on Day 1 exhibited the fluorescence of EGFP mounted on the MAC vector, and thus, it was confirmed that the CYP3A4+/7R ES cells could contribute to tissue generation. On the other hand, the fluorescence of DsRed that reflects the expression of CYP3A7 was strongly observed in the liver and the small intestine (Figure 5, liver). Hepatocytes suitable for tests of drug-metabolizing enzyme inducibility could be efficiently selected using DsRed fluorescence as an indicator.

### (2) Production of CYP3A4+/7R MAC-introduced mice:

The chimeric mice produced in Example 5(1) were subjected to breeding to obtain a CYP3A4+/7R MAC-introduced mouse strain. The expression of DsRed was confirmed in the liver of this CYP3A4+/7R mouse on 1st day after the birth (Figure 17).

### Example 6 Production of HepaRG cells, into which bacterial artificial chromosome is introduced:

### (1) Production of loxP tg-HepaRG:

A plasmid vector (VH21-12#8-3) comprising HPRT exon1-2, loxP, and a Hyg-resistant gene as a drug selection marker, was linearized by treating it with the restriction enzyme (NotI). Introduction of this vector into HepaRG and the obtainment of a hygromycin (400 µg/ml) resistant clone were carried out in the same manner as that in the production of loxP tg-HepG2. When HepaRG was subjected to a growing culture, only the growth-dedicated medium of Biopredic International (a 720 differentiation medium that was an MIL700 basic medium, to which ADD720 had been added) was used as a culture medium. In addition, upon subculture, cells were removed using 0.02% EDTA-added 0.005% trypsin solution. The composition of the culture medium of BIOPREDIC has been closed.

### (2) Introduction of CYP3A4G/7R BAC into loxP tg-HepaRG:

In order to insert the above described CYP3A4G/7R-BAC into the above described loxP tg-HepaRG by a Cre-loxP system, lipofection was carried out using Lipofectamine LTX reagent. Introduction of this vector into HepaRG and the obtainment of a neomycin (800 µg/ml G418) resistant clone were carried out in the same manner as in the production of loxP tg-HepG2. CYP3A4G/7R BAC-introduced HepaRG cells, which were a cell line in which CYP3A4G/7R BAC had been introduced into loxP tg-HepaRG, were obtained. The obtainment of a stable expression cell line was confirmed by PCR using the primer sets for amplifying the EGFP and DsRed regions of a BAC vector, as shown in Table 2, and FISH analysis (Figure 10; the short arm of human chromosome 15 or the short arm of human chromosome 16).

### Test Example 1 Test regarding drug-metabolizing enzyme inducibility (microscopic observation):

The CYP3A4G/7R BAC-introduced HepG2 cells obtained in Example 2, in which the expression level of EGFP was high and the expression level of DsRed was low, were seeded in each well of a 6-well culture dish (ϕ 35 mm) coated with Cellmatrix (Nitta Gelatin, Inc.) at a cell density of 5×10⁵cells/well. From the following day for 48 hours, 50 µM or 100 µM dexamethasone (DEX) or rifampicin (RIF) was added as a CYP-inducing drug to the cells. As a result, after addition of the CYP-inducing drug, increases in the expression levels of both DsRed and EGFP were observed in the CYP3A4G/7R BAC-introduced HepG2 cells, although the expression levels of DsRed and EGFP were low when the CYP-inducing drug was not added (Figure 6). As such, since the expression of the reporter gene was observed by addition of dexamethasone and rifampicin each having ability to induce a drug-metabolizing enzyme, it is found that the ability to induce a drug-metabolizing enzyme can be accurately evaluated using the vector of the present invention. Moreover, it is also found that cells in which the expression level of DsRed downstream of the expression control region of a fetus-specific gene is low are suitable for accurate evaluation of induction of a drug-metabolizing enzyme.

### Test Example 2 Test regarding drug-metabolizing enzyme inducibility (FACS analysis):

Increases in the numbers of DsRed-positive cells and EGFP-positive cells, which were determined when 25 µM, 50 µM or 100 µM DEX or RIF was added to the cells for 48 hours under the same culture conditions as those in Test Example 1, were examined by FACS analysis. The loxP tg-HepG2 that was a transgenic cell line before insertion of BAC produced in Example 2 was used as a negative control, and the numbers of the aforementioned positive cells relative to the number of the negative control cells defined as 1 are shown in Figure 7. An increase in the fluorescence intensity of EGFP in CYP3A4G/7R BAC-introduced HepG2 cells by addition of DEX was 2- or 3-fold that in the untreated cells, and the aforementioned increase by addition of RIF was 16-fold that in the untreated cells. At this time, DsRed fluorescence intensity was also increased by approximately 15-fold that in the untreated cells. Thus, since the expression of the reporter gene was observed by addition of dexamethasone and rifampicin each having ability to induce a drug-metabolizing enzyme, it is found that the ability to induce a drug-metabolizing enzyme can be accurately evaluated using the vector of the present invention. Moreover, it is also found that the expression of DsRed used as a second reporter gene significantly increased under strongly cytotoxic conditions in which the dose of rifampicin or dexamethasone was high, and thus that DsRed is useful as a marker for cytotoxicity.

### Test Example 3 Expression of CYP3A7 upon cell damage:

The expression of CYP3A7 during the injury of hepatocytes was examined by a method of liver regeneration after partial hepatectomy performed on 70% living liver, using a humanized CYP3A model mouse into which human CYP3A4 and CYP3A7 have been incorporated, a method of damaging the liver of the same mouse as described above by administration of a CYP-inducing drug, and a method of transplanting frozen human hepatocytes into the liver injury model mouse. In the method of liver regeneration after the partial hepatectomy, mice that were fed after 70% of the liver had been excised by ligating it with a cord were produced. Thereafter, the mice were successively subjected to dissection from Days 1 to 5 to collect the liver from each mouse, and gene expression was then examined. In the method of damaging the liver by administration of a CYP-inducing drug, the liver was excised from each mouse belonging to a PCN administration group and a corn oil administration control group, and gene expression was then examined. In the method of transplanting frozen human hepatocytes into the living mouse, a part of the humanized mouse liver was obtained from PhenixBio, and gene expression was then examined. As a result, it was confirmed that the expression of CYP3A7 was transiently increased when hepatocytes were regenerated after damage had been given to the liver.

### Test Example 4 Test regarding drug-metabolizing enzyme inducibility (Analysis of total area fluorescence intensity under fluorescence microscope):

1 µM, 10 µM, 25 µM or 100 µM RIF was added to the cells for 48 hours under the same culture conditions as those in Test Example 1, and thereafter, the total area fluorescence intensity of DsRed-positive and EGFP cells was measured under a fluorescence microscope. The loxP tg-HepG2 that was a transgenic cell line before insertion of BAC produced in Example 2 was used as a negative control, and the fluorescence intensity of the aforementioned positive cells relative to the value of the negative control defined as 1 are shown in Figure 8. The fluorescence intensity of EGFP in CYP3A4G/7R BAC-introduced HepG2 cells was increased to 35-fold that in the untreated cells. At this time, the fluorescence intensity of DsRed was also increased to approximately 10-fold that in the untreated cells. As such, since induction of the expression of the reporter gene was observed by addition of rifampicin having ability to induce a drug-metabolizing enzyme, it is found that the ability to induce a drug-metabolizing enzyme can be evaluated in a cell culture state, using the vector of the present invention. These results demonstrate that a high throughput screening-type evaluation system of evaluating induction of a drug-metabolizing enzyme can be constructed using the cells of the present invention.

### Test Example 5 Test regarding drug-metabolizing enzyme inducibility (expression analysis by quantitative RT-PCR):

When 100 µM DEX and RIF were added to the cells for 48 hours under the same culture conditions as those in Test Example 1, whether or not a correlation was observed between changes in the fluorescence intensity of DsRed and EGFP and changes in the gene expression levels of endogenous CYP3A4 and CYP3A7 genes was analyzed by quantitative RT-PCR. An untreated group was used as a negative control and the fluorescence intensity of DsRed and EGFP relative to the value of the negative control defined as 1 are shown in Figure 9. The fluorescence intensity of EGFP in CYP3A4G/7R BAC-introduced HepG2 cells was increased by treating the C87 clone with DEX to approximately 100-fold that in the untreated cells. In the case of C6, the fluorescence intensity of DsRed was also increased, as well as the fluorescence intensity of EGFP. Moreover, the expression of an endogenous CYP3A7 gene was extremely similar to a change in the expression level of DsRed. On the other hand, when compared with endogenous CYP3A4, the fold increase of expression of EGFP was higher, their induction rates were not identical, and EGFP had higher sensitivity. However, it is found that it becomes easy to evaluate the possibility that a test substance has expression inducibility.

### Test Example 6 CYP3A4/7 in the differentiation process of HepaRG cells (expression analysis by immunostaining and quantitative RT-PCR):

The CYP3A4G/7R BAC-introduced HepaRG cells obtained in Example 6 were subjected to a growing culture (Growing) for 2 weeks (2W), and the cells were then subjected to a differentiation culture (Differentiation). When the HepaRG cells were subjected to a growing culture, only the growth-dedicated medium of Biopredic International (a 710 growth medium that was an MIL700 basic medium to which ADD710 had been added) was used as a culture medium. On the other hand, when the HepaRG cells were subjected to a differentiation culture, only the differentiation-dedicated medium of Biopredic International (a 720 differentiation medium that was an MIL700 basic medium to which ADD720 had been added) was used as a culture medium. The HepaRG cells differentiate into small hepatocyte-like cells, and they appear in the upper layer of a cell population. Figure 11 shows that this hepatocyte-like cell population is strongly positive to an antibody recognizing both CYP3A4 and CYP3A7, and that the expression of either CYP3A4 or CYP3A7 is high in the cell population. Changes in the gene expression levels of endogenous CYP3A4 and CYP3A7 in the culture process of HepaRG cells were analyzed by quantitative RT-PCR. As a result, a decrease in the expression of CYP3A7 and an increase in the expression of CYP3A4 were observed together with cell differentiation in HepaRG cells on Day 14 of the growing culture, and on Days 1, 2, 3, 7, and 15 of the differentiation culture (Figure 12). Therefore, it is found that the small CYP3A4/7 antibody-positive hepatocyte-like cells appearing in the upper layer of the cell population, as shown in Figure 11, are suitable for evaluation of the drug-metabolizing enzyme inducibility that expresses CYP3A4.

### Test Example 7 Differentiation of CYP3A4G/7R BAC-introduced HepaRG cells (observation under fluorescence microscope):

The CYP3A4G/7R BAC-introduced HepaRG cell clone (C3) obtained in Example 6 was seeded in a 96-well culture dish coated with Cellmatrix, and it was then subjected to a growing culture for 14 days (G14d) and then subjected to a differentiation culture for 14 days. Figure 13 shows a decrease in the number of DsRed-positive cells and an increase in the number of EGFP-positive cells, in the CYP3A4G/7R BAC-introduced HepaRG cells on Day 5 (D5d) and Day 10 (D10d) of the differentiation. Since HepaRG cells expressed DsRed that was a reporter of fetal hepatocytes during the growth phase, these cells are fetal cells that are not suitable for evaluation of the induction of a drug-metabolizing enzyme during the growth. However, the fact that when the cells are cultured in the differentiation-dedicated medium of Biopredic International, they becomes hepatocytes that sufficiently express drug-metabolizing enzymes including CYP3A4 as a typical example, can be visualized using the expression level of the EGFP reporter as an indicator. From these results, it is found that cells suitable for evaluation of drug-metabolizing enzyme inducibility can be selected by the present invention.

### Test Example 8 Differentiation process of CYP3A4G/7R BAC-introduced HepaRG cells:

The fluorescence microphotographs of the cells used in Test Example 7, which were prepared by subjecting the CYP3A4G/7R BAC-introduced HepaRG cell clone (C3) to a growing culture for 14 days and then to a differentiation culture for 5 days (D5d), were analyzed in detail. The hepatic parenchymal cells in the fetal liver are hepatoblasts, and the hepatoblasts express CYP3A7 and do not express CYP3A4. After birth, CYP3A7 cannot be detected, and thus, CYP3A4 becomes a major P450 drug-metabolizing enzyme. In this process, whether the expression of CYP3A7 is shifted to the expression of CYP3A4 in a single cell, or whether cells expressing CYP3A4 can be produced from other stem cells that do not express CYP3A7, has not yet been clarified. As shown in Figure 14, it is found that, at first, all of the CYP3A4G/7R BAC-introduced HepaRG cells are DsRed-positive cells, but that together with differentiation, DsRed expression disappears, and EGFP-positive cells appear from some DsRed-negative cells. Using CYP3A4G/7R BAC-introduced HepaRG cells, if the developmental stage of CYP3A4-positive hepatocytes having high metabolic function were clarified, it could be applied to a method of inducing cell differentiation, which comprises producing cells suitable for evaluation of drug-metabolizing enzyme inducibility from other stem cells such as ES cells or iPS cells.

### Test Example 9 Differentiation of CYP3A4G/7R BAC-introduced HepaRG cells (Analysis of total area fluorescence intensity under fluorescence microscope):

Changes over time in the total area fluorescence intensities of EGFP and DsRed in the CYP3A4G/7R BAC-introduced HepaRG cell clone (C3) used in Test Example 7 on Days 7 (G7d) and 14 (G14d) after the cell clone had been subjected to a growing culture, and on Days 1, 3, 5, 7, 10, and 12 (D1d, D3d, D5d, D7d, D10d, and D12d) of a differentiation culture, were analyzed under a fluorescence microscope. The results are shown in Figure 15. On the same day as the aforementioned analysis, total area fluorescence intensity in the cells seeded in 6 wells on a 96-well culture dish was analyzed. As a result, the CYP3A4G/7R BAC-introduced HepaRG cells extremely stably showed a decrease in the expression of DsRed and an increase in the expression of EGFP, together with cell differentiation.

### Test Example 10 Evaluation of inducibility of CYP3A4G/7R BAC-introduced HepaRG cells (analysis by quantitative RT-PCR):

CYP3A4 expression-inducing drugs (RIF: rifampicin, DEX: dexamethasone, CLO: clotrimazole, NIF: nifedipine, and PCN: pregnenolone 16α-carbonitrile) dissolved in 1% DMSO were each added to the CYP3A4G/7R BAC-introduced HepaRG cell clone (C3) used in Test Example 7, which was subjected to a growing culture for 14 days and then to a differentiation culture for 12 days. Forty-eight hours later (corresponding to Day 14 of differentiation culture), the total area fluorescence intensities of EGFP and DsRed were analyzed under a fluorescence microscope (Figure 16). The expression of EGFP was induced by all the inducing drugs other than PCN. PCN has been known to be a CYP-inducing drug for rodents, and it does not induce CYP3A4 in humans. Thus, non-inducibility by PCN is an example of showing that the reaction in humans can be predicted with EGFP. In addition, an increase in the fluorescence intensity of DsRed was observed when CLO, NIF and PCN having high cellular damage were added, and thus, it is found that an increase in the expression of CYP3A7 becomes a guideline for evaluation of cytotoxicity. Hence, since the expression of the reporter gene was observed approximately 2-fold higher than usual by addition of dexamethasone and rifampicin having ability to induce a drug-metabolizing enzyme, it is found that such ability to induce a drug-metabolizing enzyme can be accurately evaluated if CYP3A4G/7R BAC-introduced HepaRG cells are used, as with the CYP3A4G/7R BAC-induced HepG2 cells produced in Example 2.

### Test Example 11 Liver injury test using CYP3A4+/7R MAC-introduced mice (observation under fluorescence microscope):

The CYP3A4+/7MAC-introduced mice produced in Example 5 strongly expressed DsRed for several weeks after the birth. However, when the mice were 8 weeks old, the expression of DsRed in the liver was significantly reduced. To such mice, 5% carbon tetrachloride dissolved in DMSO was administered, and thereafter, the expression of CYP3A7 upon cell damage was observed for 3 days. As a result, it is found that an increase in the suppressed fluorescence intensity of DsRed in the liver can be visualized under a fluorescence microscope (Figure 18). In the CYP3A4+/7R MAC-introduced mice, EGFP functions as a marker showing introduction of MAC in the cells.

### Test Example 12 Liver injury test using CYP3A4+/7R MAC-introduced mice (analysis by quantitative RT-PCR):

Using RNAs extracted from the livers of the CYP3A4+/7R MAC-introduced mice to which 5% carbon tetrachloride had been administered in Test Example 11 and non-administered (Day 0) mice, an increase in the expression of DsRed on Days 1, 2 and 3 after the administration was analyzed by quantitative RT-PCR (Figure 19). It is found that CYP3A7-DsRed can be used as a liver regeneration marker after hepatotoxicity, as well as an α-fetoprotein (Afp) that is a marker for immature hepatoblasts.

RT-PCR primers used for confirmation of gene expression in CYP3A4/7 BAC-introduced cells are shown in the following Table 3.

### [Table 3]

**Table 3 RT-PCR primers for confirmation of gene expression in CYP3A4/7 BAC-introduced cells**

| Set | SEQ ID NO. | Primer name | Sequence (from 5' to 3') | Product size | Gene |
|---|---|---|---|---|---|
| 1 | 36 | hqCYP3A4-3A7-F | TTCATCCAATGGACTGCATAAAT | 86 bp | CYP3A4 (NM 017460) |
| | 37 | hqCYP3A4-R | TCCCAAGTATAACACTCTACACAGACAA | | |
| 2 | 38 | hqCYP3A4-3A7-F | TTCATCCAATGTGCTGCATAAAT | 86 bp | hCYP3A7 (NM_000765) |
| | 39 | qhCYP3A7-R | TACCAAGTATAACACTCTATACAGACCA | | |
| 3 | 40 | GFP101-F | CTTCAAGGAGGACGGCAACA | 101 bp | EGFP |
| | 41 | GFP101-R | CCTTGATGCCGTTCTTCTGC | | |
| 4 | 42 | DsRed q-RT F | GAAGGGCGAGATCCACAAG | 67 bp | DsRed |
| | 43 | DsRed q-RT R | GGACTTGAACTCCACCAGGTA | | |
| 5 | 44 | hAFP-F | ACCATGAAGTGGGTGGAATC | 148 bp | AFP(NM_001134.1) |
| | 45 | hAFP-R | TGGTAGCCAGGTCAGCTAAA | | |
| 6 | 46 | hβACTIN76-F | ATTGGCAATGAGCGGTTC | 76 bp | βACTIN, ACTB (NM_001101) |
| | 47 | hβACTIN76-R | GGATGCCACAGGACTCCAT | | |

## Claims

1. A vector for producing a cell that is used for evaluation of induction of a drug-metabolizing enzyme by a test substance and cytotoxicity of the test substance, which comprises an expression control region of a fetus-specific gene, a first reporter gene, an expression control region of a drug-metabolizing enzyme gene, and a second reporter gene,
wherein the first reporter gene is located downstream of the expression control region of the fetus-specific gene, and the second reporter gene is located downstream of the expression control region of the drug-metabolizing enzyme gene, wherein the drug-metabolizing enzyme gene is CYP3A4 and the fetus-specific gene is CYP3A7.

2. The vector according to claim 1, wherein the reporter gene is detected by light signal.

3. The vector according to any one of claims 1 or 2, wherein the reporter gene encodes a fluorescent protein.

4. A cell for evaluation of induction of a drug-metabolizing enzyme by a test substance and cytotoxicity of the test substance, wherein the cell comprises the vector according to any one of claims 1 to 3.

5. The cell according to claim 4, which is derived from a hepatoma-derived cell line.

6. The cell according to claim 4 or 5, which has properties of a stem cell capable of differentiating into a mature hepatocyte.

7. The cell according to any one of claims 4 to 6, which is selected by a method comprising:
(1)a step of culturing cells containing the vector according to any one of claims 1 to 3; and
(2)a step of selecting a cell using an expression level of the first reporter gene in the cells during the culture in the step (1) as an indicator.

8. The cell according to claim 7, wherein the culture in the step (1) is a culture for allowing cells containing the vector according to any one of claims 1 to 3 to differentiate into hepatocytes.

9. A non-human animal comprising the vector according to any one of claims 1 to 3.

10. A method for in vitro evaluating induction of a drug-metabolizing enzyme by a test substance and cytotoxicity of the test substance, which comprises:
(a)a step of evaluating expression of the first reporter gene in the cell according to any one of claims 4 to 8 and then removing cells involving high expression of the first reporter gene from the cells used for evaluation, wherein the contacted test substance has cytotoxicity when the increase in the expression is observed; and
(b) a step of evaluating a change in expression of the second reporter gene in the cell according to any one of claims 4 to 8 before and after the contact of the cell according to any one of claims 4 to 8 with the test substance, comprising a step of determining that the contacted test substance has ability to induce a drug-metabolizing enzyme, when the expression of the second reporter gene is increased by the contact with the test substance,
wherein the first reporter gene is located downstream of the expression control region of the fetus-specific gene, and the second reporter gene is located downstream of the expression control region of the drug-metabolizing enzyme gene, wherein the drug-metabolizing enzyme gene is CYP3A4 and the fetus-specific gene is CYP3A7.

11. The method according to claim 10, wherein the evaluation of a change in the expression of the second reporter gene in the step (b) is based on the continuous measurement results of the expression of the second reporter gene.

12. A kit for evaluating induction of a drug-metabolizing enzyme by a test substance and cytotoxicity of the test substance, wherein the kit comprises the vector according to any one of claims 1 to 3.

13. The kit according to claim 12 further comprising a cell having properties of a stem cell capable of differentiating into a mature hepatocyte.

## Patentansprüche

1. Vektor zur Herstellung einer Zelle, die zur Bewertung der Induktion eines Arzneimittel-metabolisierenden Enzyms durch eine Testsubstanz und der Zytotoxizität der Testsubstanz verwendet wird, umfassend eine Expressionskontrollregion eines fetusspezifischen Gens, ein erstes Reportergen, eine Expressionskontrollregion eines Arzneimittel-metabolisierenden Enzymgens und ein zweites Reportergen,
wobei das erste Reportergen stromabwärts der Expressionskontrollregion des fetusspezifischen Gens liegt und das zweite Reportergen stromabwärts der Expressionskontrollregion des Arzneimittel-metabolisierenden Enzymgens liegt, wobei das Arzneimittel-metabolisierende Enzymgen CYP3A4 ist und das fetusspezifische Gen CYP3A7 ist.

2. Vektor nach Anspruch 1, wobei das Reportergen durch ein Lichtsignal nachgewiesen wird.

3. Vektor nach einem der Ansprüche 1 oder 2, wobei das Reportergen ein fluoreszierendes Protein codiert.

4. Zelle zur Bewertung der Induktion eines Arzneimittel-metabolisierenden Enzyms durch eine Testsubstanz und der Zytotoxizität der Testsubstanz, wobei die Zelle den Vektor nach einem der Ansprüche 1 bis 3 umfasst.

5. Zelle nach Anspruch 4, die von einer von Hepatomen abgeleiteten Zelllinie abgeleitet ist.

6. Zelle nach Anspruch 4 oder 5, die Eigenschaften einer Stammzelle aufweist, die in der Lage ist, sich in einen reifen Hepatozyten zu differenzieren.

7. Zelle nach einem der Ansprüche 4 bis 6, die durch ein Verfahren ausgewählt wird, umfassend:
(1) einen Schritt zum Kultivieren von Zellen, die den Vektor nach einem der Ansprüche 1 bis 3 enthalten; und
(2) einen Schritt des Auswählens einer Zelle unter Verwendung eines Expressionsniveaus des ersten Reportergens in den Zellen während der Kultur in Schritt (1) als Indikator.

8. Zelle nach Anspruch 7, wobei die Kultur in Schritt (1) eine Kultur ist, die es Zellen, die den Vektor nach einem der Ansprüche 1 bis 3 enthalten, ermöglicht, sich in Hepatozyten zu differenzieren.

9. Nicht-menschliches Tier, umfassend den Vektor nach einem der Ansprüche 1 bis 3.

10. Verfahren zur Bewertung der Induktion eines Arzneimittel-metabolisierenden Enzyms durch eine Testsubstanz und der Zytotoxizität der Testsubstanz, umfassend:
(a) einen Schritt zur Bewertung der Expression des ersten Reportergens in der Zelle nach einem der Ansprüche 4 bis 8 und anschließendes Entfernen von Zellen, die eine hohe Expression des ersten Reportergens beinhalten, aus den zur Bewertung verwendeten Zellen, wobei die kontaktierte Testsubstanz eine Zytotoxizität aufweist, wenn eine Zunahme der Expression beobachtet wird; und
(b) einen Schritt zur Bewertung einer Änderung der Expression des zweiten Reportergens in der Zelle nach einem der Ansprüche 4 bis 8 vor und nach dem Kontakt der Zelle nach einem der Ansprüche 4 bis 8 mit der Testsubstanz, umfassend einen Schritt des Bestimmens, dass die kontaktierte Testsubstanz die Fähigkeit hat, ein Arzneimittelmetabolisierendes Enzym zu induzieren, wenn die Expression des zweiten Reportergens durch den Kontakt mit der Testsubstanz erhöht wird,
wobei das erste Reportergen stromabwärts der Expressionskontrollregion des fetusspezifischen Gens liegt und das zweite Reportergen stromabwärts der Expressionskontrollregion des Arzneimittel-metabolisierenden Enzymgens liegt, wobei das Arzneimittel-metabolisierende Enzymgen CYP3A4 ist und das fetusspezifische Gen CYP3A7 ist.

11. Verfahren nach Anspruch 10, wobei die Bewertung einer Änderung der Expression des zweiten Reportergens in Schritt (b) auf den kontinuierlichen Messergebnissen der Expression des zweiten Reportergens basiert.

12. Kit zur Bewertung der Induktion eines Arzneimittel-metabolisierenden Enzyms durch eine Testsubstanz und der Zytotoxizität der Testsubstanz, wobei das Kit den Vektor nach einem der Ansprüche 1 bis 3 umfasst.

13. Kit nach Anspruch 12, weiterhin umfassend eine Zelle mit Eigenschaften einer Stammzelle, die in der Lage ist, sich in einen reifen Hepatozyten zu differenzieren.

## Revendications

1. Vecteur pour la production d'une cellule qui est utilisée pour l'évaluation de l'induction par une substance d'essai d'une enzyme métabolisant des médicaments et de la cytotoxicité de la substance d'essai, qui comprend une région régulatrice de l'expression d'un gène spécifique du foetus, un premier gène rapporteur, une région régulatrice de l'expression d'un gène d'enzyme métabolisant des médicaments, et un second gène rapporteur,
dans lequel le premier gène rapporteur est localisé en aval de la région régulatrice de l'expression du gène spécifique du foetus, et le second gène rapporteur est localisé en aval de la région régulatrice de l'expression du gène d'enzyme métabolisant des médicaments, le gène d'enzyme métabolisant des médicaments étant CYP3A4 et le gène spécifique du foetus étant CYP3A7.

2. Vecteur selon la revendication 1, dans lequel le gène rapporteur est détecté par un signal lumineux.

3. Vecteur selon l'une quelconque des revendications 1 et 2, dans lequel le gène rapporteur code une protéine fluorescente.

4. Cellule pour évaluation de l'induction par une substance d'essai d'une enzyme métabolisant des médicaments et de la cytotoxicité de la substance d'essai, dans lequel la cellule comprend le vecteur selon l'une quelconque des revendications 1 à 3.

5. Cellule selon la revendication 4, qui est issue d'une lignée cellulaire issue d'hépatome.

6. Cellule selon la revendication 4 ou 5, qui a des propriétés d'une cellule souche capable de différenciation en un hépatocyte mature.

7. Cellule selon l'une quelconque des revendications 4 à 6, qui est sélectionnée par un procédé comprenant :
(1) une étape consistant à cultiver des cellules contenant le vecteur selon l'une quelconque des revendications 1 à 3 ; et
(2) une étape consistant à sélectionner une cellule en utilisant comme un indicateur un taux d'expression du premier gène rapporteur dans les cellules au cours de la culture dans l'étape (1).

8. Cellule selon la revendication 7, dans laquelle la culture dans l'étape (1) est une culture destinée à permettre aux cellules contenant le vecteur selon l'une quelconque des revendications 1 à 3 de se différencier en hépatocytes.

9. Animal non humain comprenant le vecteur selon l'une quelconque des revendications 1 à 3.

10. Procédé pour l'évaluation in vitro de l'induction par une substance d'essai d'une enzyme métabolisant des médicaments et de la cytotoxicité de la substance d'essai, qui comprend :
(a) une étape consistant à évaluer l'expression du premier gène rapporteur dans la cellule selon l'une quelconque des revendications 4 à 8 et ensuite à séparer des cellules utilisées pour l'évaluation les cellules présentant une haute expression du premier gène rapporteur, dans lequel la substance d'essai mise en contact présente une cytotoxicité lorsque l'augmentation de l'expression est observée ; et
(b) une étape consistant à évaluer une modification de l'expression du second gène rapporteur dans la cellule selon l'une quelconque des revendications 4 à 8 avant et après la mise en contact de la cellule selon l'une quelconque des revendications 4 à 8 avec la substance d'essai, comprenant une étape consistant à déterminer que la substance d'essai mise en contact a une aptitude à induire une enzyme métabolisant des médicaments, lorsque l'expression du second gène rapporteur est augmentée par la mise en contact avec la substance d'essai,
dans lequel le premier gène rapporteur est localisé en aval de la région régulatrice de l'expression du gène spécifique du foetus, et le second gène rapporteur est localisé en aval de la région régulatrice de l'expression du gène d'enzyme métabolisant des médicaments, le gène d'enzyme métabolisant des médicaments étant CYP3A4 et le gène spécifique du foetus étant CYP3A7.

11. Procédé selon la revendication 10, dans lequel l'évaluation d'une modification dans l'expression du second gène rapporteur dans l'étape (b) est basée sur les résultats de la mesure continue de l'expression du second gène rapporteur.

12. Nécessaire destiné à l'évaluation de l'induction d'une enzyme métabolisant des médicaments par une substance d'essai et de la cytotoxicité de la substance d'essai, dans lequel le nécessaire comprend le vecteur selon l'une quelconque des revendications 1 à 3.

13. Nécessaire selon la revendication 12, comprenant encore une cellule ayant des propriétés d'une cellule souche capable de différenciation en un hépatocyte mature.
